# EUROPEAN PATENT SPECIFICATION

(11) **EP 4 382 510 B1**
(45) Date of publication and mention of the grant of the patent: **14.05.2025**
(21) Application number: 22212327.5
(22) Date of filing: 08.12.2022
(51) Int. Cl.: C07C 311/48, H10K 85/30, H10K 85/60

(54) **COMPOUND OF FORMULA (I), ORGANIC ELECTRONIC DEVICE COMPRISING A COMPOUND OF FORMULA (1), DISPLAY DEVICE COMPRISING THE ORGANIC ELECTRONIC DEVICE AS WELL AS COMPOUNDS OF FORMULA (1) FOR USE IN ORGANIC ELECTRONIC DEVICES**
VERBINDUNG DER FORMEL (I), ORGANISCHE ELEKTRONISCHE VORRICHTUNG MIT EINER VERBINDUNG DER FORMEL(1), ANZEIGEVORRICHTUNG
COMPOSÉ DE FORMULE (I), DISPOSITIF ÉLECTRONIQUE ORGANIQUE COMPRENANT UN COMPOSÉ DE FORMULE (I), DISPOSITIF D'AFFICHAGE COMPRENANT LE DISPOSITIF ÉLECTRONIQUE ORGANIQUE

(43) Date of publication of application: 12.06.2024
(73) Proprietor: Novaled GmbH, 01099 Dresden (DE)
(72) Inventor: HEGGEMANN, Ulrich, 01099 Dresden (DE); WILLMANN, Steffen, 01099 Dresden (DE); UVAROV, Vladimir, 01099 Dresden (DE); PINTER, Piermaria, 01099 Dresden (DE)
(74) Representative: Michalski Hüttermann & Partner Patentanwälte mbB

(56) References cited:
- WO-A1-2021/123064

## Description

### Technical Field

The present invention relates to a compound of formula (I), an organic electronic device comprising a compound of formula (1) and a display device comprising the organic electronic device. The invention further relates to novel compounds of formula (1) which can be of use in organic electronic devices.

### Background Art

Organic electronic devices, such as organic light-emitting diodes OLEDs, which are self-emitting devices, have a wide viewing angle, excellent contrast, quick response, high brightness, excellent operating voltage characteristics, and color reproduction. A typical OLED comprises an anode, a hole transport layer HTL, an emission layer EML, an electron transport layer ETL, and a cathode, which are sequentially stacked on a substrate. In this regard, the HTL, the EML, and the ETL are thin films formed from organic compounds.

When a voltage is applied to the anode and the cathode, holes injected from the anode move to the EML, via the HTL, and electrons injected from the cathode move to the EML, via the ETL. The holes and electrons recombine in the EML to generate excitons. When the excitons drop from an excited state to a ground state, light is emitted. The injection and flow of holes and electrons should be balanced, so that an OLED having the above-described structure has excellent efficiency and/or a long lifetime.

Performance of an organic light emitting diode may be affected by characteristics of the organic semiconductor layer, and among them, may be affected by characteristics of metal complexes which are also contained in the organic semiconductor layer. WO2021/123064 discloses organic electronic devices comprising sulfonimide-type compounds.

There remains a need to improve performance of organic semiconductor materials, semiconductor layers, as well as organic electronic devices thereof, in particular to achieve improved operating voltage, improved lifetime and/or improved operating voltage stability over time through improving the characteristics of the compounds comprised therein.

Additionally, there is a need to provide compounds with improved thermal properties.

### DISCLOSURE

An aspect of the present invention provides a compound of formula (I)

M^{n⊕}(L^{⊖})ₙ (AL)ₘ (I),

wherein
- M: is a metal ion;
- n: is the valency of M and selected from 1 to 4;
- L: is a ligand of formula (II) 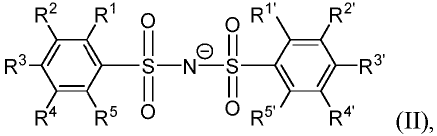
Wherein
R¹ to R⁵ and R^{1'} to R^{5'} are independently selected from substituted or unsubstituted C₁ to C₆ alkyl, halogen, Cl, F, CN, H or D;
At least one R¹ to R⁵ or R^{1'} to R^{5'} is selected from substituted C₂ to C₆ alkyl, wherein the substituent is selected from halogen, Cl, F, CN;
AL is an ancillary ligand;
m is an integer from 0 to 2.

The negative charge in the compounds of formula (I) may be delocalised partially or fully over the N(SO₂)₂ group and optionally also over the phenyl groups.

It should be noted that throughout the application and the claims any Rⁿ etc. always refer to the same moieties, unless otherwise noted.

In the present specification, when a definition is not otherwise provided, "partially fluorinated" refers to an alkyl group or an alkoxy group in which only part of the hydrogen atoms are replaced by fluorine atoms.

In the present specification, when a definition is not otherwise provided, "perfluorinated" refers to an alkyl group or an alkoxy group in which all hydrogen atoms are replaced by fluorine atoms.

In the present specification, when a definition is not otherwise provided, "substituted" refers to one substituted with a deuterium, C₁ to C₁₂ alkyl and C₁ to C₁₂ alkoxy.

However, in the present specification "aryl substituted" refers to a substitution with one or more aryl groups, which themselves may be substituted with one or more aryl and/or heteroaryl groups.

Correspondingly, in the present specification "heteroaryl substituted" refers to a substitution with one or more heteroaryl groups, which themselves may be substituted with one or more aryl and/or heteroaryl groups.

In the present specification, when a definition is not otherwise provided, an "alkyl group" refers to a saturated aliphatic hydrocarbyl group. The alkyl group may be a C₁ to C₁₂ alkyl group. More specifically, the alkyl group may be a C₁ to C₁₀ alkyl group or a C₁ to C₆ alkyl group. For example, a C₁ to C₄ alkyl group includes 1 to 4 carbons in alkyl chain, and may be selected from methyl, ethyl, propyl, iso-propyl, n-butyl, iso-butyl, sec-butyl, and tert-butyl.

Specific examples of the alkyl group may be a methyl group, an ethyl group, a propyl group, an iso-propyl group, a butyl group, an iso-butyl group, a tert-butyl group, a pentyl group, a hexyl group.

In the context of the present invention, "ⁱCₙH₍₂ₙ₊₁₎" denotes an iso-alkyl group and "ⁱCₙF₍₂ₙ₊₁₎" denotes a perfluorinated iso-alkyl group.

The term "cycloalkyl" refers to saturated hydrocarbyl groups derived from a cycloalkane by formal abstraction of one hydrogen atom from a ring atom comprised in the corresponding cycloalkane. Examples of the cycloalkyl group may be a cyclopropyl group, a cyclobutyl group, a cyclopentyl group, a cyclohexyl group, a methylcyclohexyl group, an adamantly group and the like.

The term "hetero" is understood the way that at least one carbon atom, in a structure which may be formed by covalently bound carbon atoms, is replaced by another polyvalent atom. Preferably, the heteroatoms are selected from B, Si, N, P, O, S; more preferably from N, P, O, S.

In the present specification, "aryl group" refers to a hydrocarbyl group which can be created by formal abstraction of one hydrogen atom from an aromatic ring in the corresponding aromatic hydrocarbon. Aromatic hydrocarbon refers to a hydrocarbon which contains at least one aromatic ring or aromatic ring system. Aromatic ring or aromatic ring system refers to a planar ring or ring system of covalently bound carbon atoms, wherein the planar ring or ring system comprises a conjugated system of delocalized electrons fulfilling Hückel's rule. Examples of aryl groups include monocyclic groups like phenyl or tolyl, polycyclic groups which comprise more aromatic rings linked by single bonds, like biphenyl, and polycyclic groups comprising fused rings, like naphthyl or fluorenyl.

Analogously, under heteroaryl, it is especially where suitable understood a group derived by formal abstraction of one ring hydrogen from a heterocyclic aromatic ring in a compound comprising at least one such ring.

Under heterocycloalkyl, it is especially where suitable understood a group derived by formal abstraction of one ring hydrogen from a saturated cycloalkyl ring in a compound comprising at least one such ring.

The term "fused aryl rings" or "condensed aryl rings" is understood the way that two aryl rings are considered fused or condensed when they share at least two common sp²-hybridized carbon atoms

In the present specification, the single bond refers to a direct bond.

In the context of the present invention, "different" means that the compounds do not have an identical chemical structure.

The term "free of", "does not contain", "does not comprise" does not exclude impurities which may be present in the compounds prior to deposition. Impurities have no technical effect with respect to the object achieved by the present invention.

The term "contacting sandwiched" refers to an arrangement of three layers whereby the layer in the middle is in direct contact with the two adjacent layers.

The terms "light-absorbing layer" and "light absorption layer" are used synonymously.

The terms "light-emitting layer", "light emission layer" and "emission layer" are used synonymously.

The terms "OLED", "organic light-emitting diode" and "organic light-emitting device" are used synonymously.

The terms anode, anode layer and anode electrode are used synonymously.

The terms cathode, cathode layer and cathode electrode are used synonymously.

In the specification, hole characteristics refer to an ability to donate an electron to form a hole when an electric field is applied and that a hole formed in the anode may be easily injected into the emission layer and transported in the emission layer due to conductive characteristics according to a highest occupied molecular orbital (HOMO) level.

In addition, electron characteristics refer to an ability to accept an electron when an electric field is applied and that electrons formed in the cathode may be easily injected into the emission layer and transported in the emission layer due to conductive characteristics according to a lowest unoccupied molecular orbital (LUMO) level.

The term "LUMO level" is understood to mean the lowest unoccupied molecular orbital and is determined in eV (electron volt).

The term "LUMO level further away from vacuum level" is understood to mean that the absolute value of the LUMO level is higher than the absolute value of the LUMO level of the reference compound.

The term "HOMO level" is understood to mean the highest occupied molecular orbital and is determined in eV (electron volt).

The term "HOMO level further away from vacuum level" is understood to mean that the absolute value of the HOMO level is higher than the absolute value of the HOMO level of the reference compound. For example, the term "further away from vacuum level than the HOMO level of N2,N2,N2',N2',N7,N7,N7',N7'-octakis(4-methoxyphenyl)-9,9'-spirobi[fluorene]-2,2',7,7'-tetraamine is understood to mean that the absolute value of the HOMO level of the matrix compound of the hole injection layer is higher than the HOMO level of N2,N2,N2',N2', N7,N7, N7',N7'-octakis(4-methoxyphenyl)-9,9'-spirobi[fluorene]-2,2',7,7'-tetraamine.

The term "absolute value" is understood to mean the value without the "- "symbol. According to one embodiment of the present invention, the HOMO level of the matrix compound of the hole injection layer may be calculated by quantum mechanical methods.

### Advantageous Effects

Surprisingly, it was found that the compounds according to formula (I) have improved thermal properties.

According to one embodiment, the following compounds are disclaimed: M = Ag(I) or Cu(II) and L =

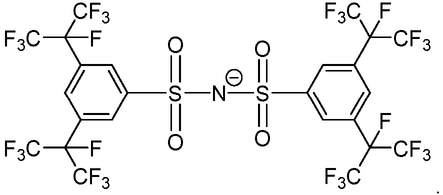

According to one embodiment, the ligand L does not include the following moiety:

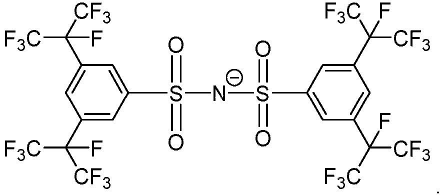

According to one embodiment of the present invention, the valency n of M of the compound of formula (I) is 1 or 2.

According to one embodiment, wherein M of the compound of formula (I) may be selected from a metal ion wherein the corresponding metal has an electronegativity value according to Allen of less than 2.4.

The term "electronegativity value according to Allen" especially refers to Allen, Leland C. (1989). "Electronegativity is the average one-electron energy of the valence-shell electrons in ground-state free atoms", Journal of the American Chemical Society 111 (25): 9003-9014.

According to one embodiment, M may be selected from an alkali metal, alkaline earth metal, transition metal, or group III or V metal.

The alkali metals may be selected from the group comprising Li, Na, K, Rb or Cs. The alkaline earth metals may be selected from the group comprising Mg, Ca, Sr or Ba. The transition metals may be selected from Sc, Y, La, Ti, Zr, V, Nb, Ta, Cr, Mo, W, Mn, Re, Fe, Co, Ni, Cu, Ag, Au or Zn. The rare earth metal may be selected from Ce. The group III and V metal may be selected from the group comprising Bi and Al.

According to one embodiment of the present invention, M is selected from Li, Na, K, Rb, Cs, Mg, Mn, Cu, Zn, Ag, Bi and Ce; preferably M is selected from Na, K, Rb, Cs, Mg, Mn, Cu, Zn, Ag and Bi; also preferred M is selected from Na, K, Rb, Cs, Mg, Mn, Cu, Zn, Ag and Bi, wherein if M is Cu, n is 2.

According to one embodiment, wherein M is selected from Li, Na, K, Rb, Cs, Mg, Ag, Ce or Bi.

According to one embodiment of the present invention, M is not Li.

According to one embodiment of the present invention M is Na or Ag.

According to one embodiment of the present invention, at least one of R¹ to R⁵ and R^{1'} to R^{5'} is perhalogenated C₂ to C₆ alkyl, preferably perhalogenated C₂ to C₃ alkyl.

According to one embodiment of the present invention, at least one of R¹ to R⁵ and R^{1'} to R^{5'} is perfluorinated C₂ to C₆ alkyl, preferably perfluorinated C₂ to C₃ alkyl.

According to one embodiment of the present invention, at least two of R¹ to R⁵ or R^{1'} to R^{5'} are selected from substituted C₂ to C₆ alkyl, wherein the substituent is selected from halogen, Cl, F, CN; preferably C₂ to C₆ perhalogenated alkyl and most preferred preferably C₂ to C₆ perfluorinated alkyl.

According to one embodiment of the present invention, at least two of R¹ to R⁵ or R^{1'} to R^{5'} are selected from substituted C₁ to C₆ alkyl, wherein the substituent is selected from halogen, Cl, F, CN; preferably C₁ to C₆ perhalogenated alkyl and most preferred preferably C₁ to C₆ perfluorinated alkyl.

According to one embodiment of the present invention, the ligand of formula (II) is selected from one of the following B1 to B4

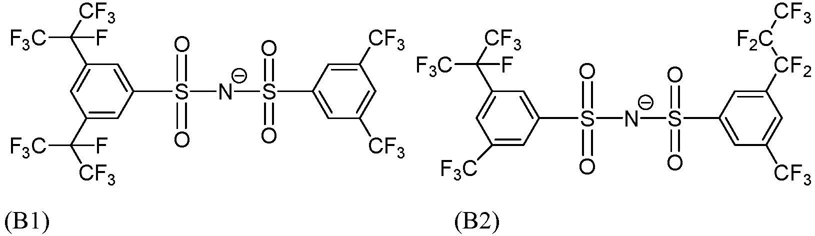

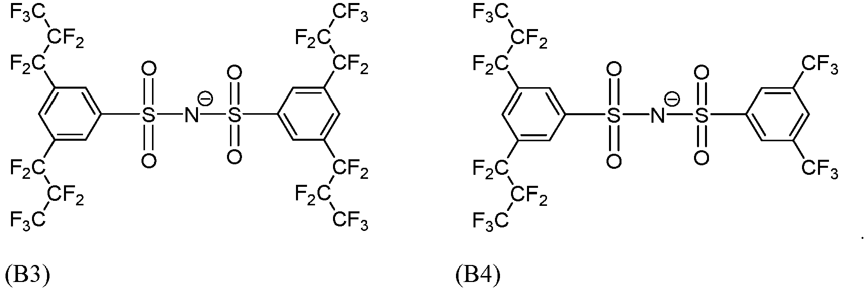

According to one embodiment of the present invention, the compound of formula (I) is selected from one of the following molecules A1 to A6:

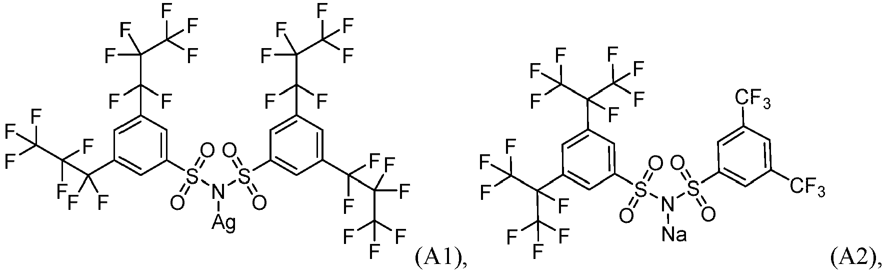

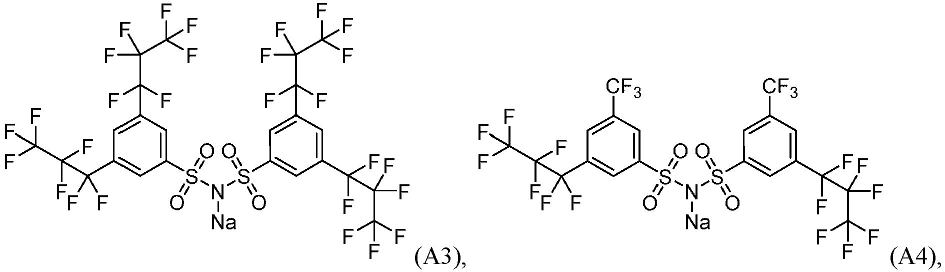

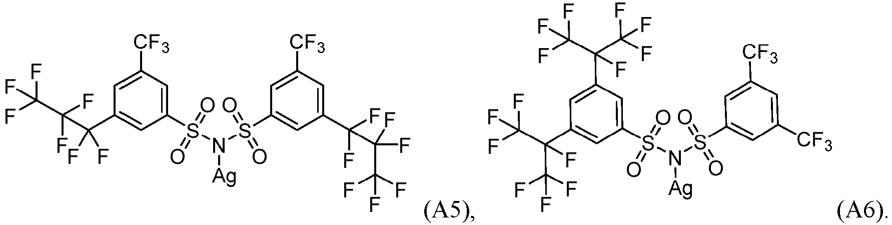

According to one embodiment of the application AL (the ancillary ligand) is selected from the group comprising H₂O, C₂ to C₄₀ mono- or multi-dentate ethers and C₂ to C₄₀ thioethers, C₂ to C₄₀ amines, C₂ to C₄₀ phosphine, C₂ to C₂₀ alkyl nitrile or C₂ to C₄₀ aryl nitrile, or a compound according to Formula (AL-I); 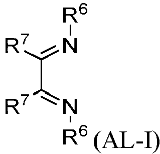 , wherein
- R⁶ and R⁷: are independently selected from C₁ to C₂₀ alkyl, C₁ to C₂₀ heteroalkyl, C₆ to C₂₀ aryl, heteroaryl with 5 to 20 ring-forming atoms, halogenated or perhalogenated C₁ to C₂₀ alkyl, halogenated or perhalogenated C₁ to C₂₀ heteroalkyl, halogenated or perhalogenated C₆ to C₂₀ aryl, halogenated or perhalogenated heteroaryl with 5 to 20 ring-forming atoms, or at least one R⁶ and R⁷ are bridged and form a 5 to 20 member ring, or the two R⁶ and/or the two R⁷ are bridged and form a 5 to 40 member ring or form a 5 to 40 member ring comprising an unsubstituted or C₁ to C₁₂ substituted phenanthroline.

### Semiconductor material

According to another aspect, a semiconductor material is provided which comprises at least one compound of formula (I) according to the present invention.

According to one embodiment, wherein the semiconductor material comprises in addition at least one covalent matrix compound or at least one substantially covalent matrix compound.

According to another aspect, wherein a semiconductor material comprises at least one compound of formula (I) according to the present invention and in addition at least one covalent matrix compound or at least one substantially covalent matrix compound.

### Organic semiconductor layer

According to another aspect, an organic semiconductor layer is provided which comprises at least one compound of formula (I) according to the present invention.

The organic semiconductor layer may be formed on the anode layer or cathode layer by vacuum deposition, spin coating, printing, casting, slot-die coating, Langmuir-Blodgett (LB) deposition, or the like. When the organic semiconductor layer is formed using vacuum deposition, the deposition conditions may vary according to the compound(s) that are used to form the layer, and the desired structure and thermal properties of the layer. In general, however, conditions for vacuum deposition may include a deposition temperature of 100° C to 350° C, a pressure of 10⁻⁸ to 10⁻³ Torr (1 Torr equals 133.322 Pa), and a deposition rate of 0.1 to 10 nm/sec.

When the organic semiconductor layer is formed using spin coating or printing, coating conditions may vary according to the compound(s) that are used to form the layer, and the desired structure and thermal properties of the organic semiconductor layer. For example, the coating conditions may include a coating speed of about 2000 rpm to about 5000 rpm, and a thermal treatment temperature of about 80° C to about 200° C. Thermal treatment removes a solvent after the coating is performed.

The thickness of the organic semiconductor layer may be in the range from about 1 nm to about 20 nm, and for example, from about 2 nm to about 15 nm, alternatively about 2 nm to about 12 nm.

When the thickness of the organic semiconductor layer is within this range, the organic semiconductor layer may have excellent hole injecting and/or hole generation characteristics, without a substantial penalty in driving voltage.

According to one embodiment of the present invention, the organic semiconductor layer may comprise:
- at least about ≥ 0.5 wt.-% to about ≤ 30 wt.-%, preferably about ≥ 0.5 wt.-% to about ≤ 20 wt.-%, and more preferred about ≥ 1 wt.-% to about ≤ 15 wt.-% of a compound of formula (I), and
- at least about ≥ 70 wt.-% to about ≤ 99.5 wt.-%, preferably about ≥ 80 wt.-% to about ≤ 99.5 wt.-%, and more preferred about ≥ 85 wt.-% to about ≤ 99 wt.-% of a substantially covalent matrix compound; preferably the wt.-% of the compound of formula (I) is lower than the wt.-% of the substantially covalent matrix compound; wherein the weight-% of the components are based on the total weight of the organic semiconductor layer.

According to one embodiment of the present invention the organic semiconductor layer and/or the compound of formula (1) are non-emissive.

In the context of the present specification the term "essentially non-emissive" or "non-emissive" means that the contribution of the compound or layer to the visible emission spectrum from the device is less than 10 %, preferably less than 5 % relative to the visible emission spectrum. The visible emission spectrum is an emission spectrum with a wavelength of about ≥ 380 nm to about ≤ 780 nm.

### Substantially covalent matrix compound/ covalent matrix compound

According to another aspect of the present inventon the semiconductor material and/or the organic semiconductor layer may further comprise a substantially covalent matrix compound.

The substantially covalent matrix compound, also named matrix compound, may be an organic aromatic matrix compounds, which comprises organic aromatic covalent bonded carbon atoms. The substantially covalent matrix compound may be an organic compound, consisting substantially from covalently bound C, H, O, N, S, which may optionally comprise also covalently bound B, P or Si. The substantially covalent matrix compound may be an organic aromatic covalent bonded compound, which is free of metal atoms, and the majority of its skeletal atoms may be selected from C, O, S, N and preferably from C, O and N, wherein the majority of atoms are C-atoms. Alternatively, the covalent matrix compound is free of metal atoms and majority of its skeletal atoms may be selected from C and N, preferably the covalent matrix compound is free of metal atoms and majority of its skeletal atoms may be selected from C and the minority of its skeletal atoms may be N.

According to one embodiment, the substantially covalent matrix compound may have a molecular weight Mw of ≥ 400 and ≤ 2000 g/mol, preferably a molecular weight Mw of ≥ 450 and ≤ 1500 g/mol, further preferred a molecular weight Mw of ≥ 500 and ≤ 1000 g/mol, in addition preferred a molecular weight Mw of ≥ 550 and ≤ 900 g/mol, also preferred a molecular weight Mw of ≥ 600 and ≤ 800 g/mol.

In one embodiment, the HOMO level of the substantially covalent matrix compound may be more negative than the HOMO level of N2,N2,N2',N2',N7,N7,N7',N7'-octakis(4-methoxyphenyl)-9,9'-spirobi[fluorene]-2,2',7,7'-tetraamine (CAS 207739-72-8) when determined under the same conditions.

In one embodiment of the present invention, the substantially covalent matrix compound may be free of alkoxy groups.

Preferably, the substantially covalent matrix compound comprises at least one arylamine moiety, alternatively a diarylamine moiety, alternatively a triarylamine moiety.

Preferably, the substantially covalent matrix compound is free of TPD or NPB.

### Compound of formula (IIIa) or a compound of formula (IIIb)

According to another aspect of the present invention, the substantially covalent matrix compound or covalent matrix compound, also referred to as matrix compound herein, may comprises at least one arylamine compound, diarylamine compound, triarylamine compound, a compound of formula (IIIa) or a compound of formula (IIIb): 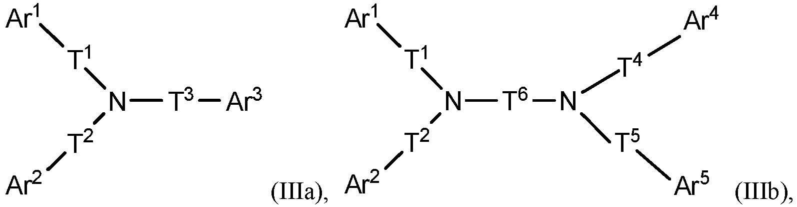 wherein:
T¹, T², T³, T⁴ and T⁵ are independently selected from a single bond, phenylene, biphenylene, terphenylene or naphthenylene, preferably a single bond or phenylene;
T⁶ is phenylene, biphenylene, terphenylene or naphthenylene;
Ar¹, Ar², Ar³, Ar⁴ and Ar⁵ are independently selected from substituted or unsubstituted C₆ to C₂₀ aryl, or substituted or unsubstituted C₃ to C₂₀ heteroarylene, substituted or unsubstituted biphenylene, substituted or unsubstituted fluorene, substituted 9-fluorene, substituted 9,9-fluorene, substituted or unsubstituted naphthalene, substituted or unsubstituted anthracene, substituted or unsubstituted phenanthrene, substituted or unsubstituted pyrene, substituted or unsubstituted perylene, substituted or unsubstituted triphenylene, substituted or unsubstituted tetracene, substituted or unsubstituted tetraphene, substituted or unsubstituted dibenzofurane, substituted or unsubstituted dibenzothiophene, substituted or unsubstituted xanthene, substituted or unsubstituted carbazole, substituted 9-phenylcarbazole, substituted or unsubstituted azepine, substituted or unsubstituted dibenzo[b,f]azepine, substituted or unsubstituted 9,9'-spirobi[fluorene], substituted or unsubstituted spiro[fluorene-9,9'-xanthene], or a substituted or unsubstituted aromatic fused ring system comprising at least three substituted or unsubstituted aromatic rings selected from the group comprising substituted or unsubstituted non-hetero, substituted or unsubstituted hetero 5-member rings, substituted or unsubstituted 6-member rings and/or substituted or unsubstituted 7-member rings, substituted or unsubstituted fluorene, or a fused ring system comprising 2 to 6 substituted or unsubstituted 5- to 7-member rings and the rings are selected from the group comprising (i) unsaturated 5- to 7-member ring of a heterocycle, (ii) 5- to 6-member of an aromatic heterocycle, (iii) unsaturated 5- to 7-member ring of a non-heterocycle, (IIIa) 6-member ring of an aromatic non-heterocycle; wherein the substituents of Ar¹, Ar², Ar³, Ar⁴ and Ar⁵ are selected the same or different from the group comprising H, D, F, C(-O)R², CN, Si(R²)₃, P(-O)(R²)₂, OR², S(-O)R², S(-O)₂R², substituted or unsubstituted straight-chain alkyl having 1 to 20 carbon atoms, substituted or unsubstituted branched alkyl having 1 to 20 carbon atoms, substituted or unsubstituted cyclic alkyl having 3 to 20 carbon atoms, substituted or unsubstituted alkenyl or alkynyl groups having 2 to 20 carbon atoms, substituted or unsubstituted substituted or unsubstituted aromatic ring systems having 6 to 40 aromatic ring atoms, and substituted or unsubstituted heteroaromatic ring systems having 5 to 40 aromatic ring atoms, unsubstituted C₆ to C₁₈ aryl, unsubstituted C₃ to C₁₈ heteroaryl, a fused ring system comprising 2 to 6 unsubstituted 5- to 7-member rings and the rings are selected from the group comprising unsaturated 5- to 7-member ring of a heterocycle, 5- to 6-member of an aromatic heterocycle, unsaturated 5- to 7-member ring of a non-heterocycle, and 6-member ring of an aromatic non-heterocycle,
   wherein R² may be selected from H, D, straight-chain alkyl having 1 to 6 carbon atoms, branched alkyl having 1 to 6 carbon atoms, cyclic alkyl having 3 to 6 carbon atoms, alkenyl or alkynyl groups having 2 to 6 carbon atoms, C₆ to C₁₈ aryl or C₃ to C₁₈ heteroaryl.

Preferably, the substituents of Ar¹, Ar², Ar³, Ar⁴ and Ar⁵ are selected the same or different from the group comprising H, straight-chain alkyl having 1 to 6 carbon atoms, branched alkyl having 1 to 6 carbon atoms, cyclic alkyl having 3 to 6 carbon atoms, alkenyl or alkynyl groups having 2 to 6 carbon atoms, C₆ to C₁₈ aryl, C₃ to C₁₈ heteroaryl, a fused ring system comprising 2 to 4 unsubstituted 5- to 7-member rings and the rings are selected from the group comprising unsaturated 5- to 7-member ring of a heterocycle, 5- to 6-member of an aromatic heterocycle, unsaturated 5- to 7-member ring of a non-heterocycle, and 6-member ring of an aromatic non-heterocycle; more preferred the substituents are selected the same or different from the group consisting of H, straight-chain alkyl having 1 to 4 carbon atoms, branched alkyl having 1 to 4 carbon atoms, cyclic alkyl having 3 to 4 carbon atoms and/or phenyl.

Thereby, the compound of formula (IIIa) or (IIIb) may have a rate onset temperature suitable for mass production.

According to an embodiment of the semiconductor material and/or organic semiconductor layer, wherein the substantially covalent matrix compound comprises a compound of formula (IIIa) or formula (IIIb): 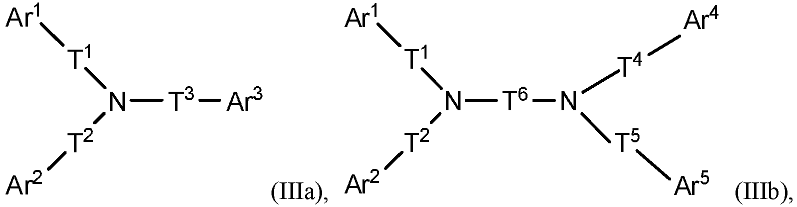 wherein
T¹, T², T³, T⁴ and T⁵ may be independently selected from a single bond, phenylene, biphenylene, terphenylene or naphthenylene, preferably a single bond or phenylene;
T⁶ is phenylene, biphenylene, terphenylene or naphthenylene;
Ar¹, Ar², Ar³, Ar⁴ and Ar⁵ may be independently selected from unsubstituted C₆ to C₂₀ aryl, or unsubstituted C₃ to C₂₀ heteroarylene, unsubstituted biphenylene, unsubstituted fluorene, substituted 9-fluorene, substituted 9,9-fluorene, unsubstituted naphthalene, unsubstituted anthracene, unsubstituted phenanthrene, unsubstituted pyrene, unsubstituted perylene, unsubstituted triphenylene, unsubstituted tetracene, unsubstituted tetraphene, unsubstituted dibenzofurane, unsubstituted dibenzothiophene, unsubstituted xanthene, unsubstituted carbazole, substituted 9-phenylcarbazole, unsubstituted azepine, unsubstituted dibenzo[b,f]azepine, unsubstituted 9,9'-spirobi[fluorene], unsubstituted spiro[fluorene-9,9'-xanthene], or a unsubstituted aromatic fused ring system comprising at least three unsubstituted aromatic rings selected from the group comprising unsubstituted non-hetero, unsubstituted hetero 5-member rings, unsubstituted 6-member rings and/or unsubstituted 7-member rings, unsubstituted fluorene, or a fused ring system comprising 2 to 6 unsubstituted 5- to 7-member rings and the rings are selected from the group comprising (i) unsaturated 5- to 7-member ring of a heterocycle, (ii) 5- to 6-member of an aromatic heterocycle, (iii) unsaturated 5- to 7-member ring of a non-heterocycle, (IIIa) 6-member ring of an aromatic non-heterocycle.

According to an embodiment of the semiconductor material and/or organic semiconductor layer, wherein the substantially covalent matrix compound comprises a compound of formula (IIIa) or formula (IIIb): 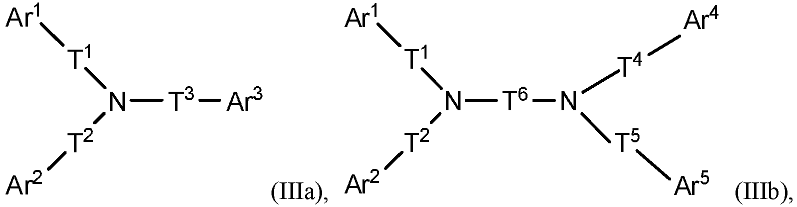 wherein
T¹, T², T³, T⁴ and T⁵ may be independently selected from a single bond, phenylene, biphenylene, terphenylene or naphthenylene, preferably a single bond or phenylene;
T⁶ is phenylene, biphenylene, terphenylene or naphthenylene;
Ar¹, Ar², Ar³, Ar⁴ and Ar⁵ may be independently selected from unsubstituted C₆ to C₂₀ aryl, or unsubstituted C₃ to C₂₀ heteroarylene, unsubstituted biphenylene, unsubstituted fluorene, substituted 9-fluorene, substituted 9,9-fluorene, unsubstituted naphthalene, unsubstituted anthracene, unsubstituted phenanthrene, unsubstituted pyrene, unsubstituted perylene, unsubstituted triphenylene, unsubstituted tetracene, unsubstituted tetraphene, unsubstituted dibenzofurane, unsubstituted dibenzothiophene, unsubstituted xanthene, unsubstituted carbazole, substituted 9-phenylcarbazole, unsubstituted azepine, unsubstituted dibenzo[b,f]azepine, unsubstituted 9,9'-spirobi[fluorene], unsubstituted spiro[fluorene-9,9'-xanthene].

Thereby, the compound of formula (IIIa) or (IIIb) may have a rate onset temperature suitable for mass production.

According to an embodiment wherein T¹, T², T³, T⁴ and T⁵ may be independently selected from a single bond, phenylene, biphenylene or terphenylene.

According to an embodiment wherein T¹, T², T³, T⁴ and T⁵ may be independently selected from phenylene, biphenylene or terphenylene and one of T¹, T², T³, T⁴ and T⁵ are a single bond. According to an embodiment wherein T¹, T², T³, T⁴ and T⁵ may be independently selected from phenylene or biphenylene and one of T¹, T², T³, T⁴ and T⁵ are a single bond.

According to an embodiment wherein T¹, T², T³, T⁴ and T⁵ may be independently selected from phenylene or biphenylene and two of T¹, T², T³, T⁴ and T⁵ are a single bond.

According to an embodiment wherein T¹, T² and T³ may be independently selected from phenylene and one of T¹, T² and T³ are a single bond.

According to an embodiment wherein T¹, T² and T³ may be independently selected from phenylene and two of T¹, T² and T³ are a single bond.

According to an embodiment wherein T⁶ may be phenylene, biphenylene, terphenylene. According to an embodiment wherein T⁶ may be phenylene.

According to an embodiment wherein T⁶ may be biphenylene. According to an embodiment wherein T⁶ may be terphenylene.

According to an embodiment wherein Ar¹, Ar², Ar³, Ar⁴ and Ar⁵ may be independently selected from B1 to B16: 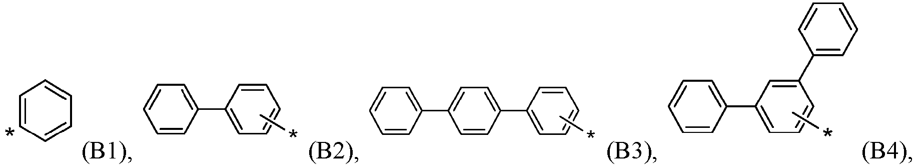 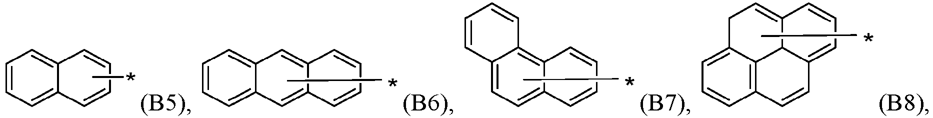 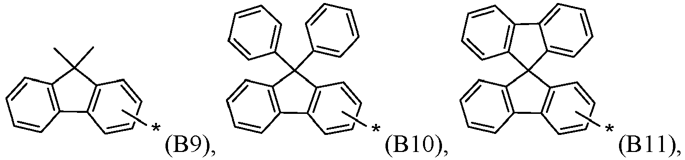 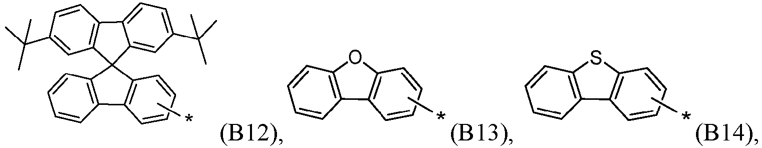 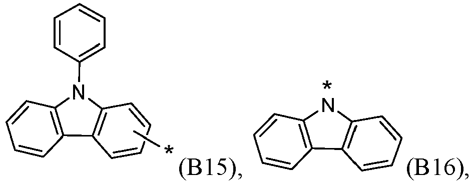 wherein the asterix "*" denotes the binding position.

According to an embodiment, wherein Ar¹, Ar², Ar³, Ar⁴ and Ar⁵ may be independently selected from B1 to B 15; alternatively selected from B1 to B10 and B13 to B 15.

According to an embodiment, wherein Ar¹, Ar², Ar³, Ar⁴ and Ar⁵ may be independently selected from the group consisting of B1, B2, B5, B7, B9, B10, B13 to B16.

The rate onset temperature may be in a range particularly suited to mass production, when Ar¹, Ar², Ar³, Ar⁴ and Ar⁵ are selected in this range.

The "matrix compound of formula (IIIa) or formula (IIIb) " may be also referred to as "hole transport compound".

According to one embodiment the compound of formula (IIIa) or formula (IIIb) may comprises at least ≥ 1 to ≤ 6 substituted or unsubstituted aromatic fused ring systems comprising heteroaromatic rings.

According to one embodiment the compound of formula (IIIa) or formula (IIIb) may comprises at least ≥ 1 to ≤ 6 substituted or unsubstituted aromatic fused ring systems comprising heteroaromatic rings and at least ≥ 1 to ≤ 3 substituted or unsubstituted unsaturated 5- to 7-member ring of a heterocycle, preferably ≥ 2 to ≤ 5 substituted or unsubstituted aromatic fused ring systems comprising heteroaromatic rings.

According to one embodiment the compound of formula (IIIa) or formula (IIIb) may comprises at least ≥ 1 to ≤ 6 substituted or unsubstituted aromatic fused ring systems comprising heteroaromatic rings and at least ≥ 1 to ≤ 3 substituted or unsubstituted unsaturated 5- to 7-member ring of a heterocycle, preferably ≥ 2 to ≤ 5 substituted or unsubstituted aromatic fused ring systems comprising heteroaromatic rings, and at least ≥ 1 to ≤ 3 substituted or unsubstituted unsaturated 5- to 7-member ring of a heterocycle, further preferred 3 or 4 substituted or unsubstituted aromatic fused ring systems comprising heteroaromatic rings and optional at least ≥ 1 to ≤ 3 substituted or unsubstituted unsaturated 5- to 7-member ring of a heterocycle, and additional preferred wherein the aromatic fused ring systems comprising heteroaromatic rings are unsubstituted and optional at least ≥ 1 to ≤ 3 unsubstituted unsaturated 5- to 7-member ring of a heterocycle.

According to one embodiment the compound of formula (IIIa) or formula (IIIb) may comprises:
- a substituted or unsubstituted aromatic fused ring systems with at least ≥ 2 to ≤ 6, preferably ≥ 3 to ≤ 5, or 4 fused aromatic rings selected from the group comprising substituted or unsubstituted non-hetero aromatic rings, substituted or unsubstituted hetero 5-member rings, substituted or unsubstituted 6-member rings and/or substituted or unsubstituted unsaturated 5-to 7- member ring of a heterocycle; or
- an unsubstituted aromatic fused ring systems with at least ≥ 2 to ≤ 6, preferably ≥ 3 to ≤ 5, or 4 fused aromatic rings selected from the group comprising unsubstituted non-hetero aromatic rings, unsubstituted hetero 5-member rings, unsubstituted 6-member rings and/or unsubstituted unsaturated 5- to 7-member ring of a heterocycle.

It should be noted here that the wording "aromatic fused ring system" may include at least one aromatic ring and at least one substituted or unsubstituted unsaturated 5- to 7- member ring. It should be noted here that the substituted or unsubstituted unsaturated 5- to 7- member ring may not be an aromatic ring.

According to one embodiment, the substantially covalent matrix compound comprises at least one naphthyl group, carbazole group, dibenzofurane group, dibenzothiophene group and/or substituted fluorenyl group, wherein the substituents are independently selected from methyl, phenyl or fluorenyl.

According to an embodiment of the present invention, wherein the compound of formula (IIIa) or formula (IIIb) are selected from F1 to F20: 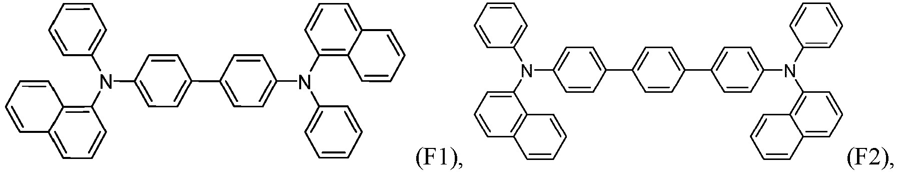 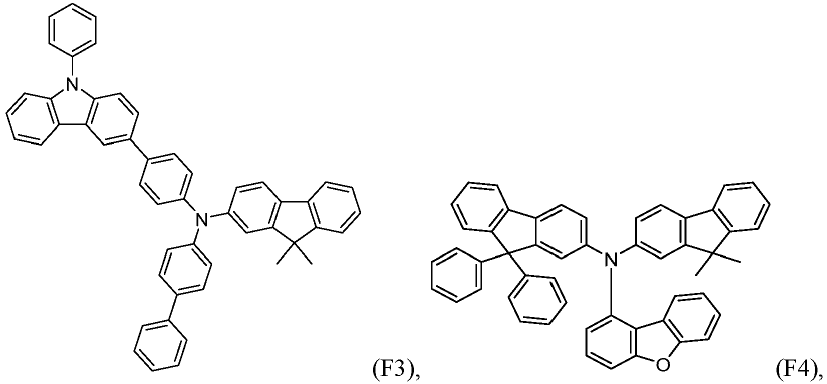 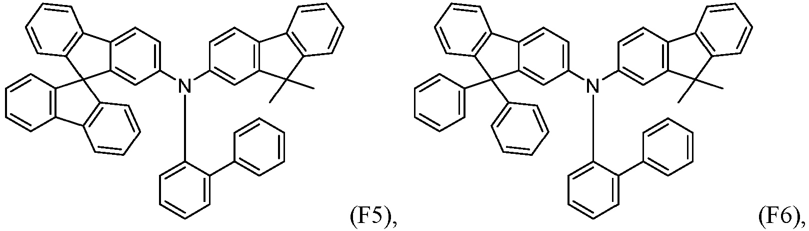 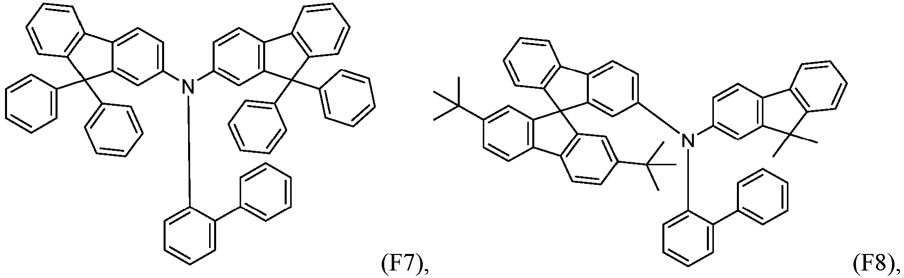 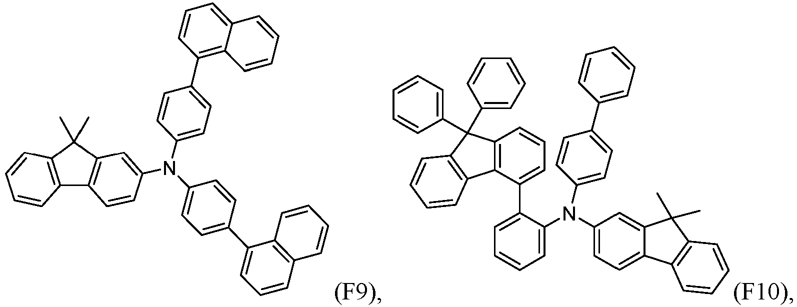 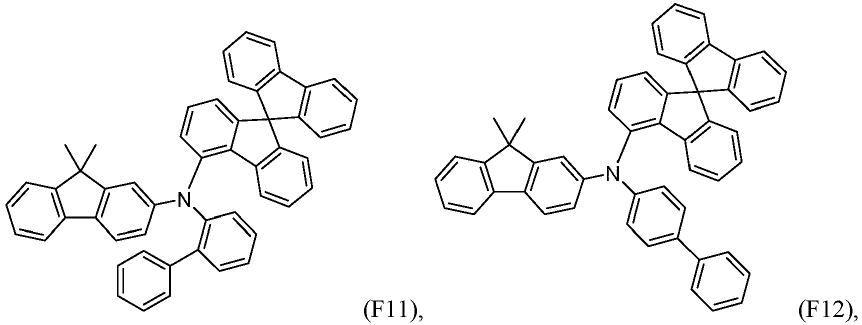 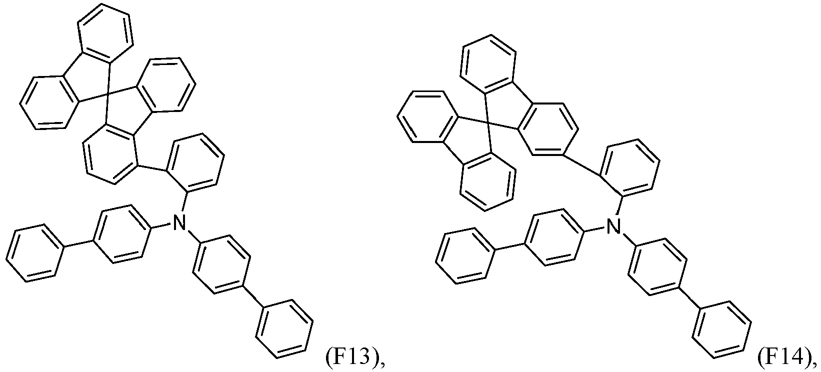 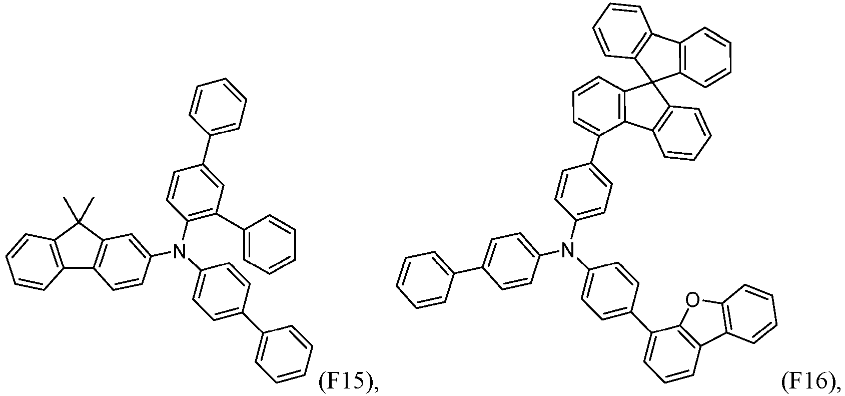 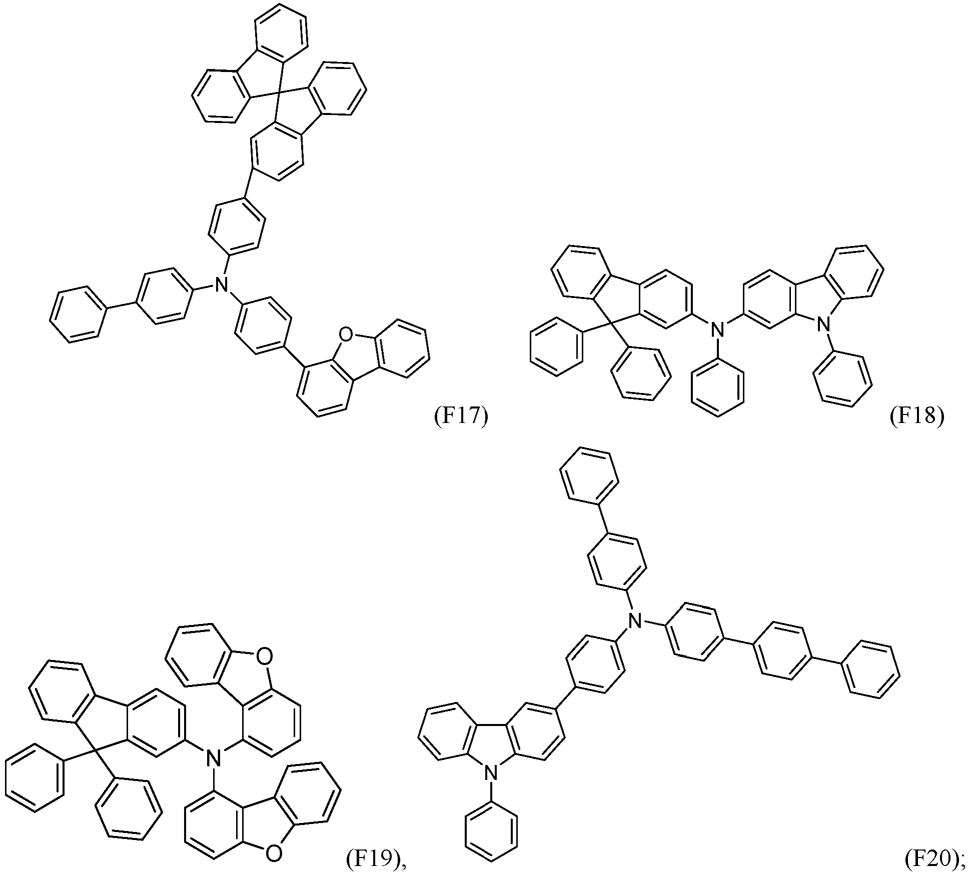 preferably the compound of formula (IIIa) or formula (IIIb) is selected from F3 to F20, more preferred F4 to F20.

The substantially covalent matrix compound may be free of HTM014, HTM081, HTM163, HTM222, EL-301, HTM226, HTM355, HTM133, HTM334, HTM604 and EL-22T. The abbreviations denote the manufacturers' names, for example, of Merck or Lumtec.

### Organic electronic device

According to another aspect of the present invention, an organic electronic device is provided, wherein the organic electronic device comprises a semiconductor material, wherein at least one semiconductor material comprises a compound of Formula (I).

According to another aspect of the present invention, an organic electronic device is provided, wherein the organic electronic device comprises an organic semiconductor layer, wherein the organic semiconductor layer comprises a compound of Formula (I).

Surprisingly it has been shown that for many applications within the present invention such an organic electronic device has improved properties, especially in view of the non-increase in operating voltage over time.

According to one embodiment of the present invention, the organic electronic device is selected from the group comprising a light emitting device, thin film transistor, a battery, a display device or a photovoltaic cell, and preferably a light emitting device, preferably the electronic device is part of a display device or lighting device.

According to one embodiment the organic electronic device comprises a compound according to Formula (I) of the present invention is a light emitting device, a thin film transistor, a battery, a display device or a photovoltaic device, and preferably a light emitting device, preferably the electronic device is part of a display device or lighting device.

According to one embodiment of the invention, the organic electronic devices further comprises at least one photoactive layer, wherein the at least one photoactive layer is arranged between the anode layer and the cathode layer.

According to one embodiment of the invention, the organic electronic devices comprises at least one photoactive layer and the at least one organic semiconductor layers is arranged between the anode and the at least one photoactive layer.

According to one embodiment the organic electronic device comprises an anode layer, a cathode layer, at least one photoactive layer and at least one semiconductor layer, wherein the at least one semiconductor layer is arranged between the anode layer and the at least one photoactive layer; and wherein the at least one organic semiconductor layer comprises a compound of Formula (1).

According to one embodiment, the organic electronic device comprises an anode layer, a cathode layer, and at least one organic semiconductor layer, wherein the at least one organic semiconductor layer is arranged between the anode layer and the cathode layer, and wherein the at least one organic semiconductor layer is the organic semiconductor layer according to the present invention.

According to one embodiment of the invention, the organic semiconductor layer is arranged and/or provided adjacent to the anode layer.

According to one embodiment of the invention, the organic semiconductor layer of the present invention is a hole-injection layer.

In case the semiconductor layer of the present invention is a hole-injection layer and/ or is arranged and/or provided adjacent to the anode layer then it is especially preferred that this layer consists essentially of the compound of formula (1).

In the context of the present specification the term "consisting essentially of " especially means and/or includes a concentration of ≥ 90% (vol/vol) more preferred ≥ 95% (vol/vol) and most preferred ≥ 99% (vol/vol).

According to another aspect, the semiconductor layer may have a layer thickness of at least about ≥ 0.5 nm to about ≤ 10 nm, preferably of about ≥ 2 nm to about ≤ 8 nm, also preferred of about ≥ 3 nm to about ≤ 5 nm.

According to one embodiment of the invention, the semiconductor layer of the present invention may further comprise a substantially covalent matrix compound. Preferably at least one semiconductor layer further comprising a substantially covalent matrix compound is arranged and/or provided adjacent to the anode layer.

According to one embodiment of the invention, the electronic organic device is an electroluminescent device, preferably an organic light emitting diode.

According to one embodiment of the invention, the electronic organic device is an electroluminescent device, preferably an organic light emitting diode and the light is emitted through the cathode layer.

The present invention furthermore relates to a display device comprising an organic electronic device according to the present invention.

According to one embodiment of the invention the electronic organic device is an electroluminescent device, preferably an organic light emitting diode.

The present invention furthermore relates to a display device comprising an organic electronic device according to the present invention.

### Further layers

In accordance with the invention, the organic electronic device may comprise, besides the layers already mentioned above, further layers. Exemplary embodiments of respective layers are described in the following:

### Substrate

The substrate may be any substrate that is commonly used in manufacturing of, electronic devices, such as organic light-emitting diodes. If light is to be emitted through the substrate, the substrate shall be a transparent or semitransparent material, for example a glass substrate or a transparent plastic substrate. If light is to be emitted through the top surface, the substrate may be both a transparent as well as a non-transparent material, for example a glass substrate, a plastic substrate, a metal substrate or a silicon substrate.

### Anode layer

The anode layer, also named anode electrode, may be formed by depositing or sputtering a material that is used to form the anode layer. The material used to form the anode layer may be a high work-function material, so as to facilitate hole injection. The anode layer may be a transparent or reflective electrode. Transparent conductive oxides, such as indium tin oxide (ITO), indium zinc oxide (IZO), tin-dioxide (SnO2), aluminum zinc oxide (AlZO) and zinc oxide (ZnO), may be used to form the anode layer. The anode layer may also be formed using metals, typically silver (Ag), gold (Au), or metal alloys.

The anode layer may comprise two or more anode sub-layers.

According to one embodiment, the anode layer comprises a first anode sub-layer and a second anode sub-layer, wherein the first anode sub-layer is arranged closer to the substrate and the second anode sub-layer is arranged closer to the cathode layer.

According to one embodiment, the anode layer may comprise a first anode sub-layer comprising or consisting of Ag or Au and a second anode-sub-layer comprising or consisting of transparent conductive oxide.

According to one embodiment, the anode layer comprises a first anode sub-layer, a second anode sub-layer and a third anode sub-layer, wherein the first anode sub-layer is arranged closer to the substrate and the second anode sub-layer is arranged closer to the cathode layer, and the third anode sub-layer is arranged between the substrate and the first anode sub-layer.

According to one embodiment, the anode layer may comprise a first anode sub-layer comprising or consisting of Ag or Au, a second anode-sub-layer comprising or consisting of transparent conductive oxide and optionally a third anode sub-layer comprising or consisting of transparent conductive oxide. Preferably the first anode sub-layer may comprise or consists of Ag, the second anode-sublayer may comprise or consists of ITO or IZO and the third anode sub-layer may comprises or consists of ITO or IZO.

Preferably the first anode sub-layer may comprise or consists of Ag, the second anode-sublayer may comprise or consists of ITO and the third anode sub-layer may comprise or consist of ITO.

Preferably, the transparent conductive oxide in the second and third anode sub-layer may be selected the same.

According to one embodiment, the anode layer may comprise a first anode sub-layer comprising Ag or Au having a thickness of 100 to 150 nm, a second anode sub-layer comprising or consisting of a transparent conductive oxide having a thickness of 3 to 20 nm and a third anode sub-layer comprising or consisting of a transparent conductive oxide having a thickness of 3 to 20 nm.

It is to be understood that the third anode layer is not part of the substrate.

According to one embodiment of the present invention, the organic semiconductor layer comprising or consisting of compound of formula (I) is in direct contact with the anode layer.

### Hole injection layer

A hole injection layer (HIL) may be formed on the anode electrode by vacuum deposition, spin coating, printing, casting, slot-die coating, Langmuir-Blodgett (LB) deposition, or the like. When the HIL is formed using vacuum deposition, the deposition conditions may vary according to the compound that is used to form the HIL, and the desired structure and thermal properties of the HIL. In general, however, conditions for vacuum deposition may include a deposition temperature of 100° C to 500° C, a pressure of 10⁻⁸ to 10⁻³ Torr (1.33322×10⁻⁶ to 1.33322×10⁻¹ Pa, as 1 Torr equals 133.322 Pa), and a deposition rate of 0.1 to 10 nm/sec.

When the HIL is formed using spin coating or printing, coating conditions may vary according to the compound that is used to form the HIL, and the desired structure and thermal properties of the HIL. For example, the coating conditions may include a coating speed of about 2000 rpm to about 5000 rpm, and a thermal treatment temperature of about 80° C to about 200° C. Thermal treatment removes a solvent after the coating is performed.

The HIL may be formed of any compound that is commonly used to form a HIL. Examples of compounds that may be used to form the HIL include a phthalocyanine compound, such as copper phthalocyanine (CuPc), 4,4',4"-tris (3-methylphenylphenylamino) triphenylamine (m-MTDATA), TDATA, 2T-NATA, polyaniline/dodecylbenzenesulfonic acid (Pani/DBSA), poly(3,4-ethylenedioxythiophene)/poly(4-styrenesulfonate) (PEDOT/PSS), polyaniline/camphor sulfonic acid (Pani/CSA), and polyaniline)/poly(4-styrenesulfonate (PANI/PSS).

The HIL may comprise or consist of p-type dopant and the p-type dopant may be selected from tetrafluoro-tetracyanoquinonedimethane (F4TCNQ), 2,2'-(perfluoronaphthalen-2,6-diylidene) dimalononitrile or 2,2',2"-(cyclopropane-1,2,3-triylidene)tris(2-(p-cyanotetrafluorophenyl)acetonitrile) but not limited hereto. The HIL may be selected from a hole-transporting matrix compound doped with a p-type dopant. Typical examples of known doped hole transport materials are: copper phthalocyanine (CuPc), which HOMO level is approximately - 5.2 eV, doped with tetrafluoro-tetracyanoquinonedimethane (F4TCNQ), which LUMO level is about -5.2 eV; zinc phthalocyanine (ZnPc) (HOMO = -5.2 eV) doped with F4TCNQ; α-NPD (N,N'-Bis(naphthalen-1-yl)-N,N'-bis(phenyl)-benzidine) doped with F4TCNQ. α-NPD doped with 2,2'-(perfluoronaphthalen-2,6-diylidene) dimalononitrile. The p-type dopant concentrations can be selected from 1 to 20 wt.-%, more preferably from 3 wt.-% to 10 wt.-%.

The thickness of the HIL may be in the range from about 1 nm to about 100 nm, and for example, from about 1 nm to about 25 nm. When the thickness of the HIL is within this range, the HIL may have excellent hole injecting characteristics, without a substantial penalty in driving voltage.

### Hole transport layer

According to one embodiment of the present invention, the organic electronic device may further comprise a hole transport layer, wherein the hole transport layer is arranged between the anode layer and the cathode layer, preferably between the organic semiconductor layer of the present invention and the cathode layer.

The hole transport layer (HTL) may be formed on the HIL by vacuum deposition, spin coating, slot-die coating, printing, casting, Langmuir-Blodgett (LB) deposition, or the like. When the HTL is formed by vacuum deposition or spin coating, the conditions for deposition and coating may be similar to those for the formation of the HIL. However, the conditions for the vacuum or solution deposition may vary, according to the compound that is used to form the HTL.

The HTL may be formed of any compound that is commonly used to form a HTL. Compounds that can be suitably used are disclosed for example in Yasuhiko Shirota and Hiroshi Kageyama, Chem. Rev. 2007, 107, 953-1010. Examples of the compound that may be used to form the HTL are: carbazole derivatives, such as N-phenylcarbazole or polyvinylcarbazole; benzidine derivatives, such as N,N'-bis(3-methylphenyl)-N,N'-diphenyl-[1,1-biphenyl]-4,4'-diamine (TPD), or N,N'-di(naphthalen-1-yl)-N,N'-diphenyl benzidine (alpha-NPD); and triphenylamine-based compound, such as 4,4',4"-tris(N-carbazolyl)triphenylamine (TCTA). Among these compounds, TCTA can transport holes and inhibit excitons from being diffused into the EML.

According to a preferred embodiment of the present invention, the hole transport layer may comprise a substantially covalent matrix compound.

According to one embodiment of the present invention, the hole transport layer may comprise the same substantially covalent matrix compound as the organic semiconductor layer of the present invention, preferably, the hole transport layer may comprise the same compound of formula (IIIa) or (IIIb) as the organic semiconductor layer of the present invention.

The thickness of the HTL may be in the range of about 5 nm to about 250 nm, preferably, about 10 nm to about 200 nm, further about 20 nm to about 190 nm, further about 40 nm to about 180 nm, further about 60 nm to about 170 nm, further about 80 nm to about 160 nm, further about 100 nm to about 160 nm, further about 120 nm to about 140 nm. A preferred thickness of the HTL may be 170 nm to 200 nm.

When the thickness of the HTL is within this range, the HTL may have excellent hole transporting characteristics, without a substantial penalty in driving voltage.

### Electron blocking layer

The function of an electron blocking layer (EBL) is to prevent electrons from being transferred from an emission layer to the hole transport layer and thereby confine electrons to the emission layer. Thereby, efficiency, operating voltage and/or lifetime may be improved. Typically, the electron blocking layer comprises a triarylamine compound. The triarylamine compound may have a LUMO level closer to vacuum level than the LUMO level of the hole transport layer. The electron blocking layer may have a HOMO level that is further away from vacuum level compared to the HOMO level of the hole transport layer. The thickness of the electron blocking layer may be selected between 2 and 20 nm.

If the electron blocking layer has a high triplet level, it may also be described as triplet control layer.

The function of the triplet control layer is to reduce quenching of triplets if a phosphorescent green or blue emission layer is used. Thereby, higher efficiency of light emission from a phosphorescent emission layer can be achieved. The triplet control layer is selected from triarylamine compounds with a triplet level above the triplet level of the phosphorescent emitter in the adjacent emission layer. Suitable compounds for the triplet control layer, in particular the triarylamine compounds, are described in EP 2 722 908 A1.

### Photoactive layer (PAL)

The photoactive layer converts an electrical current into photons or photons into an electrical current.

The PAL may be formed on the HTL by vacuum deposition, spin coating, slot-die coating, printing, casting, LB deposition, or the like. When the PAL is formed using vacuum deposition or spin coating, the conditions for deposition and coating may be similar to those for the formation of the HIL. However, the conditions for deposition and coating may vary, according to the compound that is used to form the PAL.

It may be provided that the photoactive layer does not comprise the compound of Formula (1).

The photoactive layer may be an emission layer (EML), also named light-emitting layer, or a light-absorbing layer.

### Emission layer (EML)

According to an embodiment, the organic electronic device of the present invention may further comprise an emission layer (EML), wherein the emission layer is arranged between the anode layer and the cathode layer, preferably the emission layer is arranged betweent the organic semiconductor layer and the cathode layer.

The EML may be formed on the HTL by vacuum deposition, spin coating, slot-die coating, printing, casting, LB deposition, or the like. When the EML is formed using vacuum deposition or spin coating, the conditions for deposition and coating may be similar to those for the formation of the HIL. However, the conditions for deposition and coating may vary, according to the compound that is used to form the EML.

The emission layer (EML) may comprise an organic emitter host and a light-emitting compound dopant. Examples of the organic emitter host are Alq3, 4,4'-N,N'-dicarbazole-biphenyl (CBP), poly(n-vinylcarbazole) (PVK), 9,10-di(naphthalene-2-yl)anthracene (ADN), 4,4',4"-tris(carbazol-9-yl)-triphenylamine(TCTA), 1,3,5-tris(N-phenylbenzimidazole-2-yl)benzene (TPBI), 3-tert-butyl-9,10-di-2-naphthylanthracenee (TBADN), distyrylarylene (DSA) and bis(2-(2-hydroxyphenyl)benzo-thiazolate)zinc (Zn(BTZ)2).

The emitter dopant may be a phosphorescent or fluorescent emitter. Phosphorescent emitters and emitters which emit light via a thermally activated delayed fluorescence (TADF) mechanism may be preferred due to their higher efficiency. The emitter may be a small molecule or a polymer.

Examples of red emitter dopants are PtOEP, Ir(piq)3, and Btp2lr(acac), but are not limited thereto. These compounds are phosphorescent emitters, however, fluorescent red emitter dopants could also be used.

Examples of phosphorescent green emitter dopants are Ir(ppy)3 (ppy = phenylpyridine), Ir(ppy)2(acac), Ir(mpyp)3.

Examples of phosphorescent blue emitter dopants are F2Irpic, (F2ppy)2Ir(tmd) and Ir(dfppz)3 and ter-fluorene. 4.4'-bis(4-diphenyl amiostyryl)biphenyl (DPAVBi), 2,5,8,11-tetra-tert-butyl perylene (TBPe) are examples of fluorescent blue emitter dopants.

It may be provided that the emission layer does not comprise the compound of Formula (1).

The amount of the emitter dopant may be in the range from about 0.01 to about 50 parts by weight, based on 100 parts by weight of the host. Alternatively, the emission layer may consist of a light-emitting polymer. The EML may have a thickness of about 10 nm to about 100 nm, for example, from about 20 nm to about 60 nm. When the thickness of the EML is within this range, the EML may have excellent light emission, without a substantial penalty in driving voltage.

According to a preferred embodiment of the present invention, the emission layer comprises a light-emitting compound of formula (IV): 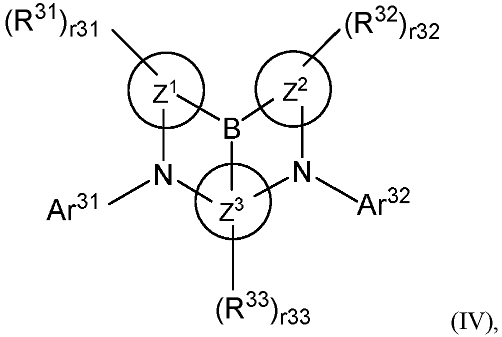 wherein
Z¹, Z² and Z³ are the same as or different from each other, and are each independently selected from the group comprising a monocyclic to polycyclic aromatic hydrocarbon ring or monocyclic to polycyclic aromatic hetero ring;
Ar³¹ and Ar³² are the same as or selected different from each other, and are each independently a substituted or unsubstituted alkyl group, a substituted or unsubstituted aryl group, or a substituted or unsubstituted heteroaryl group, or are bonded to an adjacent substituent to form a substituted or unsubstituted aromatic ring or a substituted or unsubstituted aliphatic ring;
R³¹, R³² and R³³ are the same as or different from each other, and are each independently selected from the group comprising hydrogen, deuterium, a substituted or unsubstituted alkyl group, a substituted or unsubstituted silyl group, a substituted or unsubstituted amine group, a substituted or unsubstituted aryl group, or a substituted or unsubstituted heteroaryl group, or adjacent substituents are bonded to each other to form a substituted or unsubstituted aromatic ring or a substituted or unsubstituted aliphatic ring,
wherein one or more substituents selected from the group consisting of deuterium, an alkyl group having 1 to 6 carbon atoms, an alkyl silyl group having 1 to 30 carbon atoms, an aryl silyl group having 6 to 50 carbon atoms, an alkylamine group having 1 to 30 carbon atoms, an alkylarylamine group having 1 to 50 carbon atoms, an arylamine group having 6 to 50 carbon atoms, an aryl group having 6 to 30 carbon atoms, and a heteroaryl group having 2 to 30 carbon atoms, or a substituent to which two or more substituents selected from the group are linked, or adjacent substituents are bonded to each other to form an aliphatic hydrocarbon ring having 3 to 60 carbon atoms, which is unsubstituted or substituted with the substituent;
r³¹, r³² and r³³ are each an integer from 0, 1, 2, 3 or 4, and when r³¹ to r³³ are 2 or higher, substituents in the parenthesis are the same as or different from each other.

According to one embodiment, for formula (III):
- Z¹, Z² and Z³: are the same as or selected different from each other, and are each independently selected from the group comprising a monocyclic to bicyclic aromatic hydrocarbon ring, or a monocyclic to bicyclic aromatic hetero ring containing O, N or S;
- Ar³¹ and Ar³²: are the same as or selected different from, and are each independently selected from the group comprising an alkyl group having 1 to 10 carbon atoms, which is unsubstituted or substituted with an aryl group, an aryl group having 6 to 30 carbon atoms, which is unsubstituted or substituted with an aryl group, or a heteroaryl group having 2 to 30 carbon atoms;
- R³¹, R³² and R³³: are the same as or selected different from each other, and are each independently selected from the group comprising hydrogen, deuterium, a substituted or unsubstituted alkyl group, a substituted or unsubstituted cycloalkyl group, a substituted or unsubstituted silyl group, a substituted or unsubstituted aryl group, or a substituted or unsubstituted heteroaryl group.

According to one embodiment, wherein for formula (III):
- Z¹, Z² and Z³: are the same as or selected different from each other, and are each independently selected from the group comprising a benzene ring or a thiophene ring;
- Ar³¹ and Ar³²: are the same as or selected different from each other, and are each independently selected from the group comprising phenyl group, a biphenyl group, a naphthyl group, a dimethyl fluorenyl group, a diphenyl fluorenyl group, a dibenzofuran group, or a dibenzothiophene group;
- R³¹, R³² and R³³: are the same as or selected different from each other, and are each independently selected from the group comprising hydrogen, deuterium, a substituted or unsubstituted alkyl group having 1 to 10 carbon atoms, a substituted or unsubstituted cycloalkyl group having 5 to 30 carbon atoms, a substituted or unsubstituted silyl group having 1 to 10 carbon atoms, a substituted or unsubstituted aryl group having 6 to 30 carbon atoms, or a substituted or unsubstituted heteroaryl group having 2 to 30 carbon atoms.

According to a preferred embodiment of the present invention, the emission layer comprises a light-emitting compound of formula (IV) is selected from the compounds BD1 to BD9:

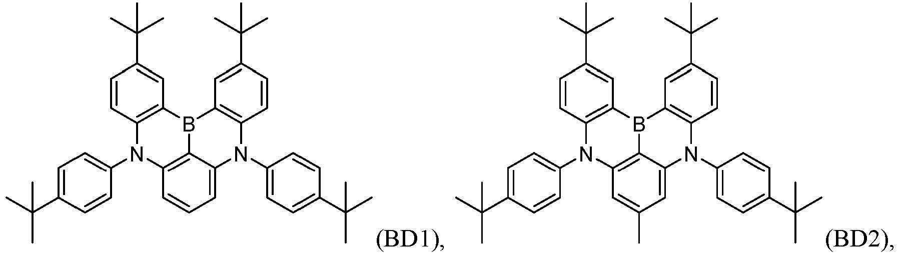

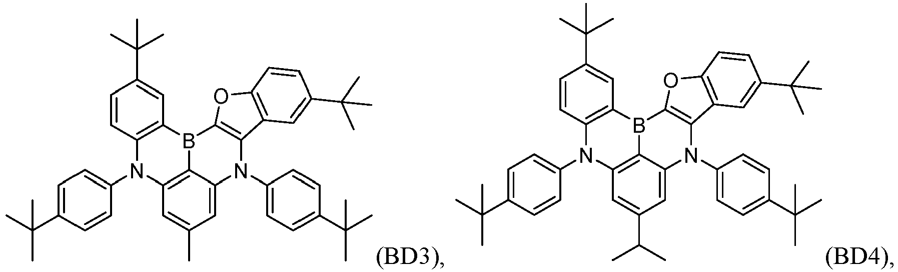

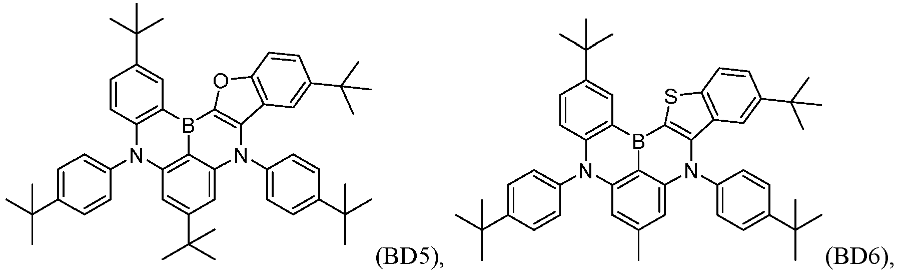

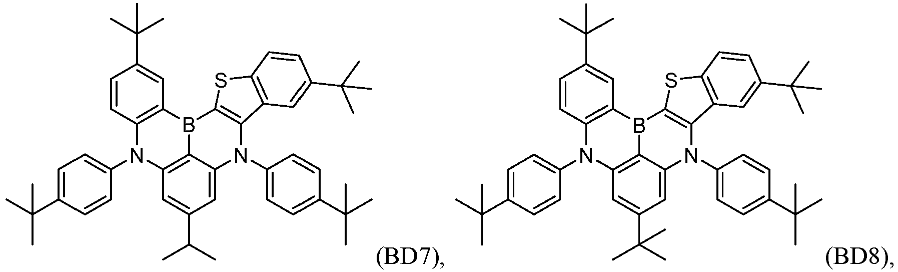

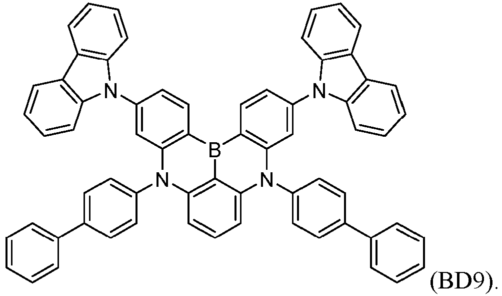

According to a preferred embodiment of the present invention, the emission layer comprises an organic emitter host compound, wherein the organic emitter host compound comprises
- at least one condensed aromatic ring system consisting of 3 to 5 rings, and
- 3 to 7 aromatic or heteroaromatic rings, wherein one or more sub-groups of the aromatic and/or heteroaromatic rings may be condensed to form fused aromatic or heteroaromatic ring systems;
wherein the molecular weight Mw of the organic emitter host compound is in the range of ≥ 400 and ≤ 2000 g/mol.

According to a preferred embodiment of the present invention, the organic emitter host compound has the formula (V) 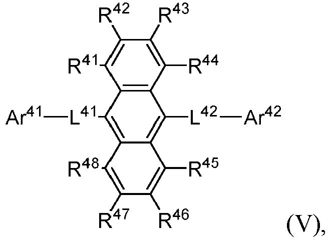 , wherein
Ar⁴¹ and Ar⁴² are independently selected from substituted or unsubstituted C₆ to C₂₄ aryl, substituted or unsubstituted C₃ to C₂₄ heteroaryl;
L⁴¹ and L⁴² are independently selected from a direct bond or substituted or unsubstituted C₆ to C₂₄ arylene, substituted or unsubstituted C₃ to C₂₄ heteroarylene;
R⁴¹ to R⁴⁸ are independently selected from H, D, substituted or unsubstituted C₁ to C₁₂ alkyl, substituted or unsubstituted C₆ to C₁₉ aryl, substituted or unsubstituted C₃ to C₁₂ heteroaryl; wherein
the substituents on Ar⁴¹, Ar⁴², L⁴¹, L⁴², R⁴¹ to R⁴⁸ are independently selected from D, C₆ to C₁₀ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy, halogen, F or CN.

According to a preferred embodiment of the present invention, the organic emitter host and/or compound of formula (V) is selected from the compounds BH1 to BH13:

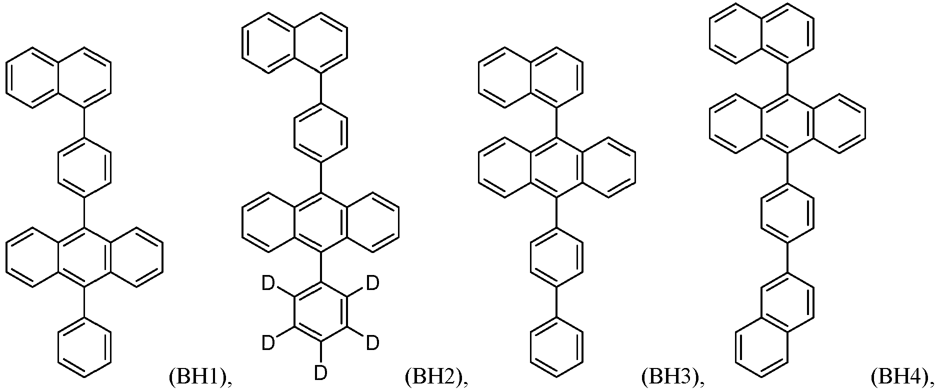

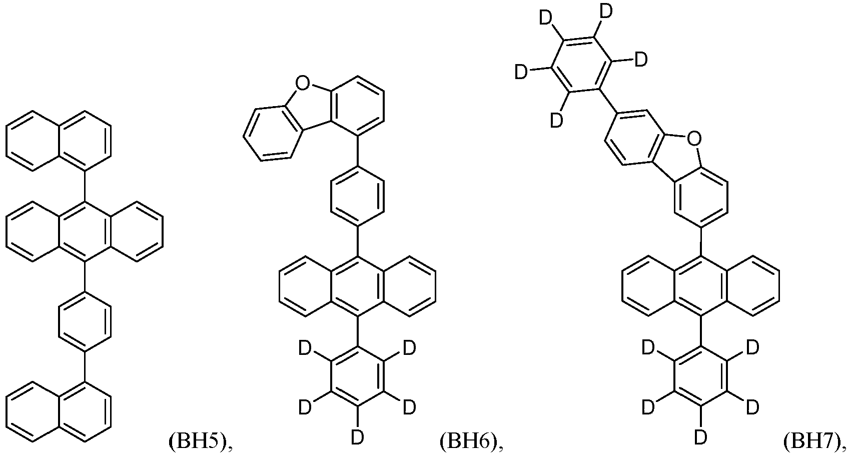

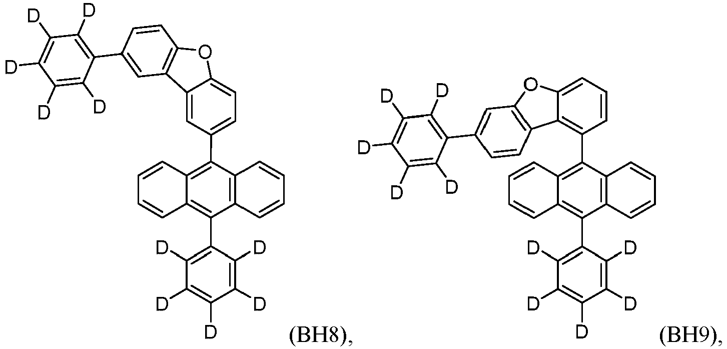

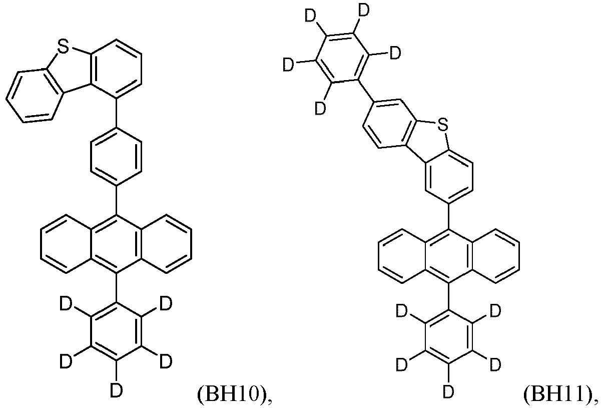

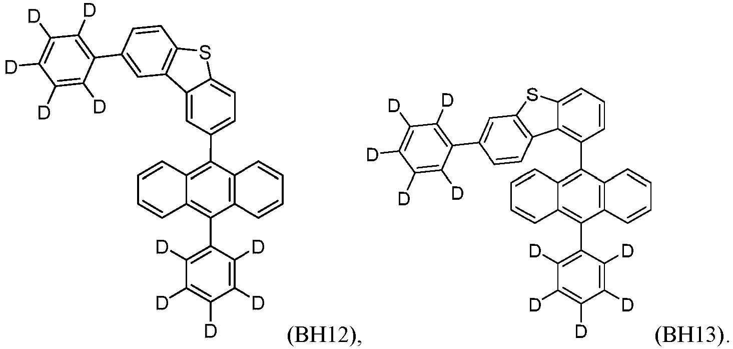

According to a preferred embodiment of the present invention, the emission layer comprises a light-emitting dopant of formula (IV) and an organic emitter host of formula (V).

According to a preferred embodiment of the present invention, the organic semiconductor layer comprises a compound of formula (I) and a compound of formula (IIIa) or formula (IIIb), the hole transport layer comprises a compound of formula (IIIa) or formula (IIIb), preferably the organic semiconductor layer and the hole transport layer comprise the same compound of formula (IIIa) or formula (IIIb) and the emission layer comprises a light-emitting dopant of formula (IV) and an organic emitter host of formula (V);
wherein the organic semiconductor layer is arranged between the anode layer and the hole transport layer, the hole transport layer is arranged between the organic semiconductor layer and the emission layer, and the emission layer is arranged between the hole transport layer and the cathode layer.

According to a preferred embodiment of the present invention, the organic semiconductor layer comprises a compound of formula (I) and a compound of formula (IIIa) or formula (IIIb), the hole transport layer comprises a compound of formula (IIIa) or formula (IIIb), preferably the organic semiconductor layer and the hole transport layer comprise the same compound of formula (IIIa) or formula (IIIb) and the emission layer comprises a light-emitting dopant of formula (IV) and an organic emitter host of formula (V);
wherein the organic semiconductor layer is arranged between the anode layer and the hole transport layer, the hole transport layer is arranged between the organic semiconductor layer and the emission layer, and the emission layer is arranged between the hole transport layer and the cathode layer;
wherein the anode layer may comprise a first anode sub-layer comprising Ag or Au having a thickness of 100 to 150 nm, a second anode sub-layer comprising or consisting of a transparent conductive oxide having a thickness of 3 to 20 nm and a third anode sub-layer comprising or consisting of a transparent conductive oxide having a thickness of 3 to 20 nm, preferably the transparent conductive oxide is selected from ITO or IZO.

### Hole blocking layer (HBL)

A hole blocking layer (HBL) may be formed on the EML, by using vacuum deposition, spin coating, slot-die coating, printing, casting, LB deposition, or the like, in order to prevent the diffusion of holes into the ETL. When the EML comprises a phosphorescent dopant, the HBL may have also a triplet exciton blocking function.

The HBL may also be named auxiliary ETL or α-ETL.

When the HBL is formed using vacuum deposition or spin coating, the conditions for deposition and coating may be similar to those for the formation of the HIL. However, the conditions for deposition and coating may vary, according to the compound that is used to form the HBL. Any compound that is commonly used to form a HBL may be used. Examples of compounds for forming the HBL include oxadiazole derivatives, triazole derivatives, phenanthroline derivatives and triazine derivatives.

The HBL may have a thickness in the range from about 5 nm to about 100 nm, for example, from about 10 nm to about 30 nm. When the thickness of the HBL is within this range, the HBL may have excellent hole-blocking properties, without a substantial penalty in driving voltage.

### Electron transport layer (ETL)

The organic electronic device according to the present invention may further comprise an electron transport layer (ETL), wherein the electron transport layer is arranged between the anode layer and the cathode layer, preferably between the organic semiconductor layer and the cathode layer.

According to another embodiment of the present invention, the electron transport layer may further comprise an azine compound, preferably a triazine compound.

In one embodiment, the electron transport layer may further comprise a dopant selected from an alkali organic complex, preferably LiQ.

The thickness of the ETL may be in the range from about 15 nm to about 50 nm, for example, in the range from about 20 nm to about 40 nm. When the thickness of the EIL is within this range, the ETL may have satisfactory electron-injecting properties, without a substantial penalty in driving voltage.

According to another embodiment of the present invention, the organic electronic device may further comprise a hole blocking layer and an electron transport layer, wherein the hole blocking layer and the electron transport layer comprise an azine compound. Preferably, the azine compound is a triazine compound.

### Electron injection layer (EIL)

An optional EIL, which may facilitates injection of electrons from the cathode, may be formed on the ETL, preferably directly on the electron transport layer. Examples of materials for forming the EIL include lithium 8-hydroxyquinolinolate (LiQ), LiF, NaCl, CsF, Li2O, BaO, Ca, Ba, Yb, Mg which are known in the art. Deposition and coating conditions for forming the EIL are similar to those for formation of the HIL, although the deposition and coating conditions may vary, according to the material that is used to form the EIL.

The thickness of the EIL may be in the range from about 0.1 nm to about 10 nm, for example, in the range from about 0.5 nm to about 9 nm. When the thickness of the EIL is within this range, the EIL may have satisfactory electron-injecting properties, without a substantial penalty in driving voltage.

### Cathode electrode

The cathode electrode is formed on the ETL or optional EIL. The cathode electrode may be formed of a metal, an alloy, an electrically conductive compound, or a mixture thereof. The cathode electrode may have a low work function. For example, the cathode electrode may be formed of lithium (Li), magnesium (Mg), aluminum (Al), aluminum (Al)-lithium (Li), calcium (Ca), barium (Ba), ytterbium (Yb), magnesium (Mg)-indium (In), magnesium (Mg)-silver (Ag), or the like. Alternatively, the cathode electrode may be formed of a transparent conductive oxide, such as ITO or IZO.

The thickness of the cathode electrode may be in the range from about 5 nm to about 1000 nm, for example, in the range from about 10 nm to about 100 nm. When the thickness of the cathode electrode is in the range from about 5 nm to about 50 nm, the cathode electrode may be transparent or semitransparent even if formed from a metal or metal alloy.

It is to be understood that the cathode electrode is not part of an electron injection layer or the electron transport layer.

### Organic light-emitting diode (OLED)

The organic electronic device according to the invention may be an organic light-emitting device.

According to one aspect of the present invention, there is provided an organic light-emitting diode (OLED) comprising: a substrate; an anode layer formed on the substrate; an organic semiconductor layer comprising compound of formula (I), a hole transport layer, an emission layer, an electron transport layer and a cathode electrode.

According to another aspect of the present invention, there is provided an OLED comprising: a substrate; an anode layer formed on the substrate; an organic semiconductor layer comprising a compound of formula (I), a hole transport layer, an electron blocking layer, an emission layer, a hole blocking layer, an electron transport layer and a cathode layer.

According to another aspect of the present invention, there is provided an OLED comprising: a substrate; an anode layer formed on the substrate; an organic semiconductor layer comprising a compound of formula (I), a hole transport layer, an electron blocking layer, an emission layer, a hole blocking layer, an electron transport layer, an electron injection layer, and a cathode layer.

According to various embodiments of the present invention, there may be provided OLEDs layers arranged between the above mentioned layers, on the substrate or on the top layer.

### Organic electronic device

The organic electronic device according to the invention may be a light emitting device, or a photovoltaic cell, and preferably a light emitting device.

According to another aspect of the present invention, there is provided a method of manufacturing an organic electronic device, the method using:
- at least one deposition source, preferably two deposition sources and more preferred at least three deposition sources.

The methods for deposition that can be suitable comprise:
- deposition via vacuum thermal evaporation;
- deposition via solution processing, preferably the processing is selected from spin-coating, printing, casting; and/or
- slot-die coating.

According to various embodiments of the present invention, there is provided a method using:
- a first deposition source to release the compound of formula (I) according to the invention, and
- a second deposition source to release the substantially covalent matrix compound; the method comprising the steps of forming the organic semiconductor layer; whereby for an organic light-emitting diode (OLED):
- the organic semiconductor layer is formed by releasing the compound of formula (I) according to the invention from the first deposition source and the substantially covalent matrix compound from the second deposition source.

According to various embodiments of the present invention, the method may further include forming on the anode layer, at least one layer selected from the group consisting of forming a hole transport layer or forming a hole blocking layer, and an emission layer between the anode layer and the first electron transport layer.

According to various embodiments of the present invention, the method may further include the steps for forming an organic light-emitting diode (OLED), wherein
- on a substrate an anode layer is formed,
- on the anode layer an organic semiconductor layer comprising a compound of formula (I) is formed,
- on the organic semiconductor layer comprising a compound of formula (I) a hole transport layer is formed,
- on the hole transport layer an emission layer is formed,
- on the emission layer an electron transport layer is formed, optionally a hole blocking layer is formed on the emission layer,
- and finally a cathode layer is formed,
- optional a hole blocking layer is formed in that order between the first anode layer and the emission layer,
- optional an electron injection layer is formed between the electron transport layer and the cathode layer.

According to various embodiments, the OLED may have the following layer structure, wherein the layers having the following order:
anode layer, organic semiconductor layer comprising a compound of formula (I) according to the invention, first hole transport layer, second hole transport layer, emission layer, optional hole blocking layer, electron transport layer, optional electron injection layer, and cathode layer.

According to another aspect of the invention, it is provided an electronic device comprising at least one organic light emitting device according to any embodiment described throughout this application, preferably, the electronic device comprises the organic light emitting diode in one of embodiments described throughout this application. More preferably, the electronic device is a display device.

Hereinafter, the embodiments are illustrated in more detail with reference to examples. However, the present disclosure is not limited to the following examples. Reference will now be made in detail to the exemplary aspects.

### Description of the Drawings

The aforementioned components, as well as the claimed components and the components to be used in accordance with the invention in the described embodiments, are not subject to any special exceptions with respect to their size, shape, material selection and technical concept such that the selection criteria known in the pertinent field can be applied without limitations.

Additional details, characteristics and advantages of the object of the invention are disclosed in the dependent claims and the following description of the respective figures which in an exemplary fashion show preferred embodiments according to the invention. Any embodiment does not necessarily represent the full scope of the invention, however, and reference is made therefore to the claims and herein for interpreting the scope of the invention. It is to be understood that both the foregoing general description and the following detailed description are exemplary and explanatory only and are intended to provide further explanation of the present invention as claimed.

### Figures 1 to 6

FIG. 1 is a schematic sectional view of an organic electronic device, according to an exemplary embodiment of the present invention;
FIG. 2 is a schematic sectional view of an organic light-emitting diode (OLED), according to an exemplary embodiment of the present invention;
FIG. 3 is a schematic sectional view of an organic light-emitting diode (OLED), according to an exemplary embodiment of the present invention.
FIG. 4 is a schematic sectional view of an organic light-emitting diode (OLED), according to an exemplary embodiment of the present invention;
FIG. 5 is a schematic sectional view of an organic light-emitting diode (OLED), according to an exemplary embodiment of the present invention.
FIG. 6 is a schematic sectional view of an organic light-emitting diode (OLED), according to an exemplary embodiment of the present invention.

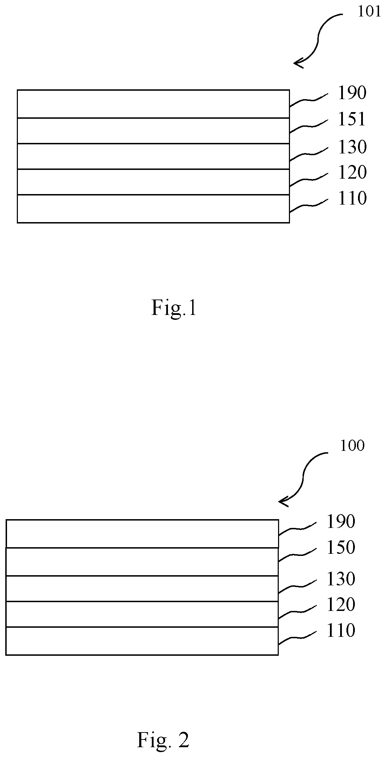

Hereinafter, the figures 1 to 6 are illustrated in more detail with reference to examples. However, the present disclosure is not limited to the following figures.

Herein, when a first element is referred to as being formed or disposed "on" or "onto" a second element, the first element can be disposed directly on the second element, or one or more other elements may be disposed there between. When a first element is referred to as being formed or disposed "directly on" or "directly onto" a second element, no other elements are disposed there between.

FIG. 1 is a schematic sectional view of an organic electronic device 101, according to an exemplary embodiment of the present invention. The organic electronic device 101 includes a substrate (110), an anode layer (120), an organic semiconductor layer comprising a compound of Formula (I) (130), a photoactive layer (PAL) (151) and a cathode layer (190).

FIG. 2 is a schematic sectional view of an organic light-emitting diode (OLED) 100, according to an exemplary embodiment of the present invention. The OLED 100 includes a substrate (110), an anode layer (120), an organic semiconductor layer comprising a compound of Formula (I) (130), an emission layer (EML) (150) and a cathode layer (190).

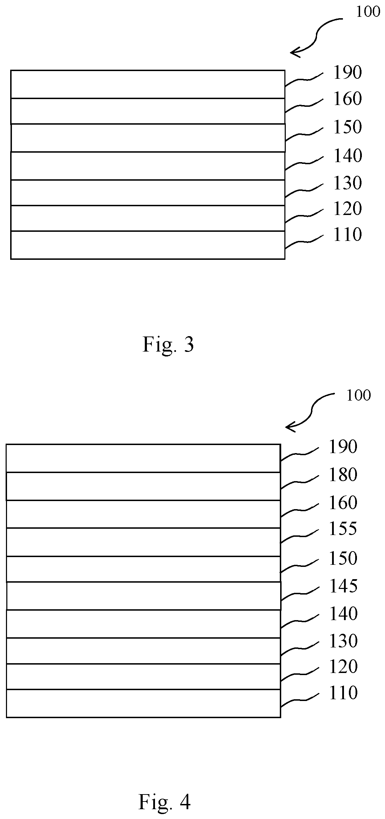

FIG. 3 is a schematic sectional view of an organic light-emitting diode (OLED) 100, according to an exemplary embodiment of the present invention. The OLED 100 includes a substrate (110), an anode layer (120), an organic semiconductor layer comprising a compound of Formula (I) (130), a hole transport layer (HTL) (140), an emission layer (EML) (150), an electron transport layer (ETL) (160) and a cathode layer (190).

FIG. 4 is a schematic sectional view of an organic light-emitting diode (OLED) 100, according to an exemplary embodiment of the present invention. The OLED 100 includes a substrate (110), an anode layer (120), an organic semiconductor layer comprising a compound of Formula (I) (130), a hole transport layer (HTL) (140), an electron blocking layer (EBL) (145), an emission layer (EML) (150), a hole blocking layer (HBL) (155), an electron transport layer (ETL) (160), an optional electron injection layer (EIL) (180), and a cathode layer (190).

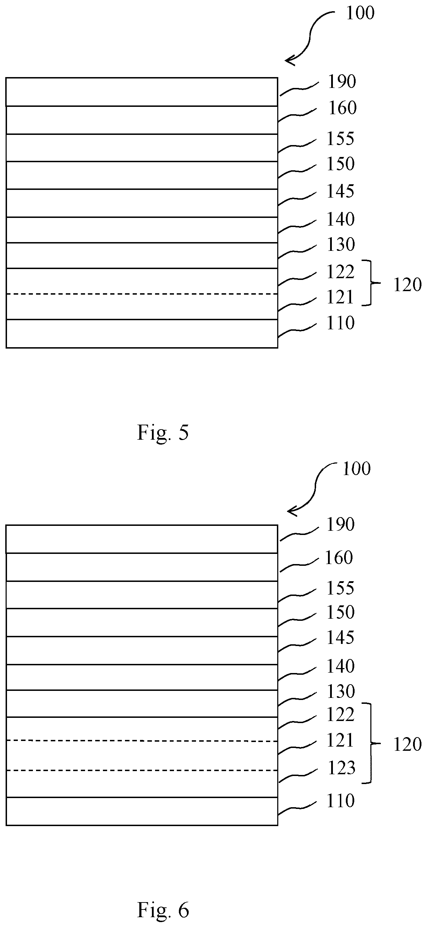

FIG. 5 is a schematic sectional view of an organic light-emitting diode (OLED) 100, according to an exemplary embodiment of the present invention. The OLED 100 includes a substrate (110), an anode layer (120) that comprises a first anode sub-layer (121) and a second anode sub-layer (122), an organic semiconductor layer comprising compound of Formula (I) (130), a hole transport layer (HTL) (140), an electron blocking layer (EBL) (145), an emission layer (EML) (150), a hole blocking layer (EBL) (155), an electron transport layer (ETL) (160) and a cathode layer (190).

FIG. 6 is a schematic sectional view of an organic light-emitting diode (OLED) 100, according to an exemplary embodiment of the present invention. The OLED 100 includes a substrate (110), an anode layer (120) that comprises a first anode sub-layer (121), a second anode sub-layer (122) and a third anode sub-layer (123), an organic semiconductor layer comprising compound of Formula (I) (130), a hole transport layer (HTL) (140), an electron blocking layer (EBL) (145), an emission layer (EML) (150), a hole blocking layer (EBL) (155), an electron transport layer (ETL) (160) and a cathode layer (190). The layers are disposed exactly in the order as mentioned before.

In the description above the method of manufacture an organic electronic device 101 of the present invention is for example started with a substrate (110) onto which an anode layer (120) is formed, on the anode layer (120), an organic semiconductor layer comprising compound of Formula (I) (130), a photoactive layer (151) and a cathode electrode 190 are formed, exactly in that order or exactly the other way around.

In the description above the method of manufacture an OLED 100 of the present invention is started with a substrate (110) onto which an anode layer (120) is formed, on the anode layer (120), an organic semiconductor layer comprising compound of Formula (I) (130), optional a hole transport layer (140), optional an electron blocking layer (145), an emission layer (150), optional a hole blocking layer (155), optional an electron transport layer (160), optional an electron injection layer (180), and a cathode electrode 190 are formed, exactly in that order or exactly the other way around.

The organic semiconductor layer comprising a compound of Formula (I) (130) can be a hole injection layer.

While not shown in Fig. 1 to Fig. 6, a capping layer and/or a sealing layer may further be formed on the cathode electrodes 190, in order to seal the OLEDs 100. In addition, various other modifications may be applied thereto.

Hereinafter, one or more exemplary embodiments of the present invention will be described in detail with, reference to the following examples. However, these examples are not intended to limit the purpose and scope of the one or more exemplary embodiments of the present invention.

### Detailed description

The invention is furthermore illustrated by the following examples which are illustrative only and non-binding.

Compounds of formula (I) may be prepared as described in WO2017029370A1 and WO2018150050A1.

### HOMO and LUMO of compounds of formula (III), (IV) and (V)

The HOMO and LUMO of compounds of formula (III), (IV) and (V) are calculated with the program package TURBOMOLE V6.5 (TURBOMOLE GmbH, Litzenhardtstrasse 19, 76135 Karlsruhe, Germany). The optimized geometries and the HOMO and LUMO energy levels of the molecular structures are determined by applying the hybrid functional B3LYP with a 6-31G* basis set in the gas phase. If more than one conformation is viable, the conformation with the lowest total energy is selected. HOMO valued for compounds of formula (III) are shown in Table 2.

Under these conditions, the HOMO level of N2,N2,N2',N2',N7,N7,N7',N7'-octakis(4-methoxyphenyl)-9,9'-spirobi[fluorene]-2,2',7,7'-tetraamine is -4.27 eV.

### HV-TGA 5% mass loss temperature

10 mg compound are loaded into 2 ccm Al₂O₃ crucible, which is mounted in high vacuum - thermogravimetric analysis (HV-TGA) setup. The HV-TGA setup consists of an evaporation source (Creaphys DE-2-CF40), a thermocouple (Thermo Sensor GmbH NiCr-Ni, Typ K) placed inside the crucible and a quartz crystal microbalance (QCM, Inficon 750-1000-G10, 6 MHz). The HV-TGA setup is part of vacuum chamber system equipped with a scroll pump, a turbomolecular pump, a nitrogen inlet with mass flow controller and a progressive valve between scroll and turbomolecular pump. The combination of nitrogen inlet and pump valve allows pressures of 1e 2 mbar to 1e-6 mbar, whereas standard operational pressure is 1e-4 mbar with a stability of +/- 10%. Subsequent to reaching desired pressure the evaporation source temperature is ramped from room temperature to 600 °C at a rate of 10 °C/min. The compound is fully evaporated and detected by the QCM. The frequency shift of the QCM during the whole temperature ramp corresponds to a mass loss of 100%.

The reference temperature for compounds of formula (I) and comparative compounds is taken at a mass loss of 5%, see Table 1, as the obtained values have closest match to processing temperature in linear evaporation sources in mass production of organic electronic devices.

### Rate onset temperature

The rate onset temperature (T_{RO}) is determined by loading 100 mg compound into a VTE source. As VTE source a point source for organic materials may be used as supplied by Kurt J. Lesker Com-pany (www.lesker.com) or CreaPhys GmbH (http://www.creaphys.com). The VTE source is heated at a constant rate of 15 K/min at a pressure of less than 10⁻⁵ mbar and the temperature inside the source measured with a thermocouple. Evaporation of the compound is detected with a QCM detector which detects deposition of the compound on the quartz crystal of the detector. The deposition rate on the quartz crystal is measured in Ǻngstrom per second. To determine the rate onset temperature, the deposition rate is plotted against the VTE source temperature. The rate onset is the temperature at which noticeable deposition on the QCM detector occurs. For accurate results, the VTE source is heated and cooled three time and only results from the second and third run are used to determine the rate onset temperature.

To achieve good control over the evaporation rate of a compound, the rate onset temperature may be in the range of 200 to 300 °C. If the rate onset temperature is below 200 °C the evaporation may be too rapid and therefore difficult to control. If the rate onset temperature is above 300 °C the evaporation rate may be too low which may result in low tact time and decomposition of the metal complex in VTE source may occur due to prolonged exposure to elevated temperatures.

The rate onset temperature is an indirect measure of the volatility of a compound. The higher the rate onset temperature the lower is the volatility of a compound.

In Table 1 are shown rate onset temperatures T_{RO} for compounds of formula (I) and comparative compounds.

### General procedure for fabrication of OLEDs

For all inventive and comparative examples (see Table 3), a glass substrate with an anode layer comprising a first anode sub-layer of 120 nm Ag, a second anode sub-layer of 8 nm ITO and a third anode sub-layer of 10 nm ITO was cut to a size of 50 mm x 50 mm x 0.7 mm, ultrasonically washed with water for 60 minutes and then with isopropanol for 20 minutes. The liquid film was removed in a nitrogen stream, followed by plasma treatment to prepare the anode layer. The plasma treatment was performed in an atmosphere comprising 97.6 vol.-% nitrogen and 2.4 vol.-% oxygen.

Then, a hole injection layer was formed on the anode layer in vacuum. Compound of formula (I) and a substantially covalent matrix compound are co-deposited on the anode layer to form a hole injection layer having a thickness of 10 nm. The composition of the hole injection layer can be seen in Table 3.

Then, a substantially covalent matrix compound was vacuum deposited on the HIL, to form a first hole transport layer (HTL). The composition and thickness of the HTL can be seen in Table 3.

Then, the electron blocking layer (EBL) was formed on the HTL having a thickness of 5 nm by depositing N,N-di([1,1'-biphenyl]-4-yl)-3'-(9H-carbazol-9-yl)-[1,1'-biphenyl]-4-amine.

Then the emission layer (EML) was formed on the EBL having a thickness of 20 nm by co-depositing 99 vol.-% EML host compound BH9 and 1 vol.-% EML dopant BD8.

Then a hole blocking layer (HBL) having a thickness of 5 nm was formed on the first emission layer by depositing 2,4-diphenyl-6-(4',5',6'-triphenyl-[1,1':2',1":3",1‴:3‴,1ʺʺ-quinquephenyl]-3ʺʺ-yl)-1,3,5-triazine (CAS 2032364-64-8).

Then, the electron transport layer (ETL) having a thickness of 32 nm was formed on the hole blocking layer by co-depositing 50 vol.-% 2-(2',6'-diphenyl-[1,1':4',1"-terphenyl]-4-yl)-4-phenyl-6-(3-(pyridin-4-yl)phenyl)-1,3,5-triazine and 50 vol.-% LiQ.

Then the electron injection layer (EIL) was formed on the electron transport layer by depositing 2 nm Yb.

Then the cathode layer having a thickness of 13 nm was formed on the electron injection layer by depositing Ag:Mg (90:10 vol.-%) at a rate of 0.01 to 1 Å/s at 10⁻⁷ mbar.

Then, N-([1,1'-biphenyl]-4-yl)-9,9-dimethyl-N-(4-(9-phenyl-9H-carbazol-3-yl)phenyl)-9H-fluoren-2-amine was deposited on the cathode layer to form a capping layer with a thickness of 75 nm.

The OLED stack is protected from ambient conditions by encapsulation of the device with a glass slide. Thereby, a cavity is formed, which includes a getter material for further protection.

To assess the performance of the inventive examples compared to the prior art, the current efficiency is measured at 20°C. The current-voltage characteristic is determined using a Keithley 2635 source measure unit, by sourcing a voltage in V and measuring the current in mA flowing through the device under test. The voltage applied to the device is varied in steps of 0.1V in the range between 0V and 10V. The operating voltage U is recorded at 10 mA/cm².

### Technical Effect of the invention

In Table 1 are shown the physical properties of compounds of formula (I) and of comparative compounds.

**Table 1: Compounds of formula (I), comparative compounds and their physical properties**

| | Chemical formula | HV-TGA 5% [°C] | T_{RO} [°C] |
|---|---|---|---|
| Comparative compound 1 CC-1 | 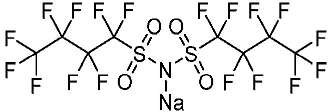 | 281 | 258 |
| Comparative compound 2 CC-2 | 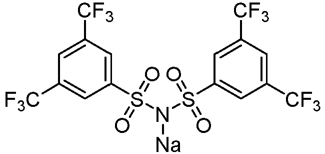 | 284 | 270 |
| Comparative compound 3 CC-3 | 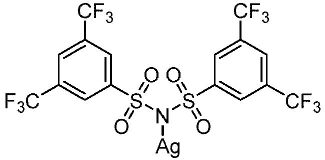 | 290 | 262 |
| MC-1 | 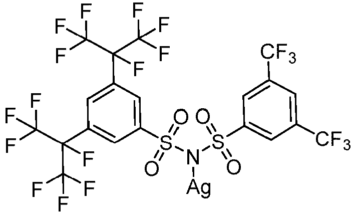 | 255 | 240 |
| MC-2 | 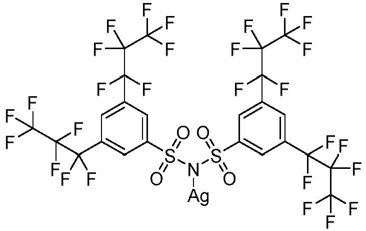 | 239 | 220 |

As can be seen in Table 1, the temperature is reduced at which 5% mass loss occurs, as determined by HV-TGA compared to comparative compounds CC-1, CC-2 and CC-3. Additionally, the rate onset temperature is in the range suitable for mass production of organic electronic devices.

For mass production of organic electronic devices, it is important that the volatility of compounds of formula (I), as quantified by HV-TGA 5% and T_{RO}, is in a range suitable for the deposition rate typically used. If the volatility is too low the desired deposition rate may not be achievable without significant decomposition of compounds of formula (I) in the vacuum thermal evaporation (VTE) source. If the volatility is too high the deposition rate may be difficult to control.

In Table 2 are shown the chemical formulas and HOMO values of a range of substantially covalent matrix compounds which may be suitably used as matrix materials in an organic semiconductor layer comprising a matrix compound and compound of formula (I).

**Table 2: Substantially covalent matrix compounds**

| Referred to as: | Chemical formula | HOMO (eV) |
|---|---|---|
| F3 | 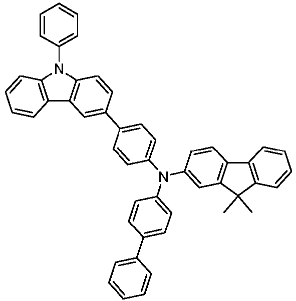 | -4.69 |
| F18 | 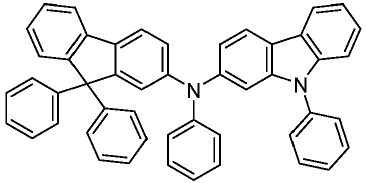 | -4.73 |
| F4 | 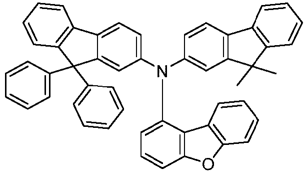 | -4.85 |
| F2 | 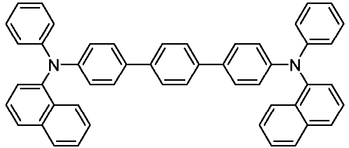 | -4.81 |

In Table 3 is shown the performance of an organic electroluminescent device comprising an organic semiconductor layer comprising compound of formula (I) and a matrix compound.

In examples 1 to 6, the organic semiconductor layer comprises compound of formula (I) MC-1 and matrix compound F18 at various concentrations. The operating voltage U is in the range of 3.52 V to 3.38 V. As can be seen in Table 3, the operating voltage is reduced with increased amount of MC-1 in the organic semiconductor layer.

In examples 7 to 12, the organic semiconductor layer comprises compound of formula (I) MC-2 and matrix compound F18. The operating voltage U is in the range of 3.49 V to 3.38 V. As can be seen in Table 3, the operating voltage is reduced with increased amount of MC-2 in the organic semiconductor layer.

In comparative examples 1 and 2, the organic semiconductor layer comprises comparative compound CC-1 and matrix compound F18. The operating voltage U is in the range of 3.58 and 3.50 V.

In comparative examples 3 and 4, the organic semiconductor layer comprises comparative compound CC-2 and matrix compound F18. The operating voltage U is in the range of 3.53 and 3.45 V.

In comparative examples 5 and 6, the organic semiconductor layer comprises comparative compound CC-3 and matrix compound F18. The operating voltage U is in the range of 3.48 and 3.45 V.

In examples 13 to 18, the organic semiconductor layer comprises compounds of formula (I) MC-1 or MC-2 and matrix compound F4. Matrix compound F4 has a HOMO level further away from vacuum level than matrix compound F18, see Table 2. The operating voltage U is in the range of 3.46 and 3.31 V.

In comparative examples 7 and 8, the organic semiconductor layer comprises comparative compound CC-3 and matrix compound F4. The operating voltage is in the range of 3.39 to 3.33 V. In summary, the increased amount of non-conductive groups in compounds of formula (I) compared to comparative compounds CC-1, CC-2 and CC-3 does not have a detrimental effect on the operating voltage of an organic electronic device.

Surprisingly, an operating voltage suitable for commercial products can be obtained even when the HOMO of the matrix compound is further away from vacuum level compared to N2,N2,N2',N2',N7,N7,N7',N7'-octakis(4-methoxyphenyl)-9,9'-spirobi[fluorene]-2,2',7,7'-tetraamine.

Without being bound by theory, a HOMO level of the matrix compound further away from vacuum level may be important for efficient hole transport into the HOMO of high efficiency emitters, for example compounds of formula (IV).

**Table 3: Performance of an organic electroluminescent device comprising an organic semiconductor layer comprising a compound of formula (I) or a comparative compound**

| | Compound of formula (I) or comparative compound in the HIL | Percentage of compound of formula (1) or comparative compound in the HIL [wt.-%] | Matrix compound in the HIL | Matrix compound in the HTL | Thickness first HTL [nm] | Voltage U at 10 mA/cm² [V] |
|---|---|---|---|---|---|---|
| Example 1 | MC-1 | 1.9 | F18 | F18 | 128 | 3.52 |
| Example 2 | MC-1 | 2.9 | F18 | F18 | 128 | 3.47 |
| Example 3 | MC-1 | 3.9 | F18 | F18 | 128 | 3.45 |
| Example 4 | MC-1 | 6.2 | F18 | F18 | 128 | 3.41 |
| Example 5 | MC-1 | 8.1 | F18 | F18 | 128 | 3.39 |
| Example 6 | MC-1 | 10.1 | F18 | F18 | 128 | 3.38 |
| Example 7 | MC-2 | 2 | F18 | F18 | 128 | 3.49 |
| Example 8 | MC-2 | 2.9 | F18 | F18 | 128 | 3.44 |
| Example 9 | MC-2 | 4 | F18 | F18 | 128 | 3.41 |
| Example 10 | MC-2 | 5.9 | F18 | F18 | 128 | 3.39 |
| Example 11 | MC-2 | 7.7 | F18 | F18 | 128 | 3.39 |
| Example 12 | MC-2 | 9.9 | F18 | F18 | 128 | 3.38 |
| Comparative example 1 | CC-1 | 6 | F18 | F18 | 128 | 3.58 |
| Comparative example 2 | CC-1 | 7.9 | F18 | F18 | 128 | 3.50 |
| Comparative example 3 | CC-2 | 2 | F18 | F18 | 128 | 3.53 |
| Comparative example 4 | CC-2 | 4 | F18 | F18 | 128 | 3.45 |
| Comparative example 5 | CC-3 | 1.9 | F18 | F18 | 128 | 3.48 |
| Comparative example 6 | CC-3 | 3.9 | F18 | F18 | 128 | 3.45 |
| Example 13 | MC-1 | 3.1 | F4 | F4 | 129 | 3.46 |
| Example 14 | MC-1 | 5 | F4 | F4 | 129 | 3.37 |
| Example 15 | MC-1 | 10.3 | F4 | F4 | 129 | 3.31 |
| Example 16 | MC-2 | 3 | F4 | F4 | 129 | 3.36 |
| Example 17 | MC-2 | 5 | F4 | F4 | 129 | 3.32 |
| Example 18 | MC-2 | 9.9 | F4 | F4 | 129 | 3.31 |
| Comparative example 7 | CC-3 | 3.1 | F4 | F4 | 129 | 3.39 |
| Comparative example 8 | CC-3 | 5 | F4 | F4 | 129 | 3.33 |

## Claims

1. A compound of Formula (1):
M^{n⊕}(L^{⊖})ₙ (AL)ₘ (I),
wherein:
M is a metal ion;
n is the valency of M and selected from 1 to 4;
L is a ligand of formula (II) 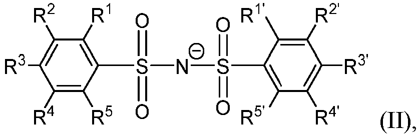
Wherein
R¹ to R⁵ and R^{1'} to R^{5'} are independently selected from substituted or unsubstituted C₁ to C₆ alkyl, halogen, Cl, F, CN, H or D;
At least one R¹ to R⁵ or R^{1'} to R^{5'} is selected from substituted C₂ to C₆ alkyl, wherein the substituent is selected from halogen, Cl, F, CN;
AL is an ancillary ligand;
m is an integer from 0 to 2.

2. The compound of claim 1, whereby the compound of formula (I) does not comprise compounds with: M = Ag(I) or Cu(II) and L =

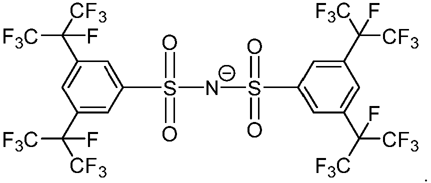

3. The compound of claim 1 or 2, whereby the compound of formula (I) does not comprise compounds whereby ligand L is

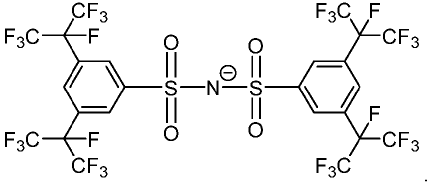

4. The compound of any of the claims 1 to 3, whereby at least one of R¹ to R⁵ and R^{1'} to R^{5'} is perhalogenated C₂ to C₆ alkyl.

5. The compound of any of the claims 1 to 4, whereby at least two of R¹ to R⁵ or R^{1'} to R^{5'} are selected from substituted C₂ to C₆ alkyl, wherein the substituent is selected from halogen, Cl, F, CN.

6. The compound of the claims 1 to 5, whereby at least two of R¹ to R⁵ or R^{1'} to R^{5'} are selected from substituted C₁ to C₆ alkyl, wherein the substituent is selected from halogen, Cl, F, CN.

7. The compound of any of the claims 1 to 6, whereby M is selected from an alkali, alkaline earth, rare earth or transition metal, alternatively M is selected from alkali, alkaline earth, transition or a group III metal.

8. An organic semiconductor layer, whereby the organic semiconductor layer comprises a compound of formula (I) of any of the preceding claims 1 to 7.

9. An organic electronic device comprising an anode layer, a cathode layer, and at least one organic semiconductor layer, wherein the at least one organic semiconductor layer is arranged between the anode layer and the cathode layer, and wherein the at least one organic semiconductor layer is the organic semiconductor layer according to claim 8.

10. The organic electronic device according to claim 9, whereby the anode layer comprises at least a first anode sub-layer and a second anode sub-layer.

11. The organic electronic device of claim 9 or 10, whereby the organic electronic device further comprises at least one photoactive layer, wherein the at least one photoactive layer is arranged between the anode layer and the cathode layer; preferably the photoactive layer is arranged between the organic semiconductor layer and the cathode layer.

12. The organic electronic device according to any of the claims 9 to 11, whereby the photoactive layer is an emission layer.

13. The organic electronic device of any of the claims 9 to 12, whereby the device comprises at
least one emission layer comprising a light-emitting compound of formula (IV) 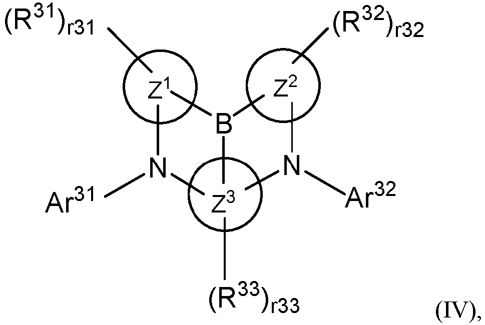 wherein
Z¹, Z² and Z³ are the same as or different from each other, and are each independently selected from the group comprising a monocyclic to polycyclic aromatic hydrocarbon ring or monocyclic to polycyclic aromatic hetero ring;
Ar³¹ and Ar³² are the same as or selected different from each other, and are each independently a substituted or unsubstituted alkyl group, a substituted or unsubstituted aryl group, or a substituted or unsubstituted heteroaryl group, or are bonded to an adjacent substituent to form a substituted or unsubstituted aromatic ring or a substituted or unsubstituted aliphatic ring;
R³¹, R³² and R³³ are the same as or different from each other, and are each independently selected from the group comprising hydrogen, deuterium, a substituted or unsubstituted alkyl group, a substituted or unsubstituted silyl group, a substituted or unsubstituted amine group, a substituted or unsubstituted aryl group, or a substituted or unsubstituted heteroaryl group, or adjacent substituents are bonded to each other to form a substituted or unsubstituted aromatic ring or a substituted or unsubstituted aliphatic ring,
wherein one or more substituents selected from the group consisting of deuterium, an alkyl group having 1 to 6 carbon atoms, an alkyl silyl group having 1 to 30 carbon atoms, an aryl silyl group having 6 to 50 carbon atoms, an alkylamine group having 1 to 30 carbon atoms, an alkylarylamine group having 1 to 50 carbon atoms, an arylamine group having 6 to 50 carbon atoms, an aryl group having 6 to 30 carbon atoms, and a heteroaryl group having 2 to 30 carbon atoms, or a substituent to which two or more substituents selected from the group are linked, or adjacent substituents are bonded to each other to form an aliphatic hydrocarbon ring having 3 to 60 carbon atoms, which is unsubstituted or substituted with the substituent; r³¹, r³² and r³³ are each an integer from 0, 1, 2, 3 or 4, and when r³¹ to r³³ are 2 or higher, substituents in the parenthesis are the same as or different from each other.

14. The organic electronic device of any of the claims 9 to 13, whereby the organic electronic device is an electroluminescent device, an organic light emitting diode (OLED), a light emitting device, thin film transistor, a battery, a display device or an organic photovoltaic cell (OPV).

15. A display device comprising an organic electronic device according to any of the claims 9 to 14.

## Patentansprüche

1. Verbindung der Formel (1):
M^{n⊕}(L^{⊖})ₙ (AL)ₘ (I),
wobei:
M für ein Metallion steht;
n für die Wertigkeit von M steht und aus 1 bis 4 ausgewählt ist;
L für einen Liganden der Formel (II) 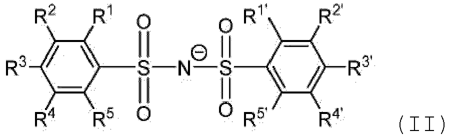
steht, wobei
R¹ bis R⁵ und R^{1'} bis R^{5'} unabhängig aus substituiertem oder unsubstituiertem C₁- bis C₆-Alkyl, Halogen, Cl, F, CN, H oder D ausgewählt sind;
mindestens ein R¹ bis R⁵ oder R^{1'} bis R^{5'} aus substituiertem C₂- bis C₆-Alkyl ausgewählt ist, wobei der Substituent aus Halogen, Cl, F, CN ausgewählt ist;
AL für einen Hilfsliganden steht;
m für eine ganze Zahl von 0 bis 2 steht.

2. Verbindung nach Anspruch 1, wobei die Verbindung der Formel (I) keine Verbindungen mit M = Ag(I) oder Cu(II) und L = 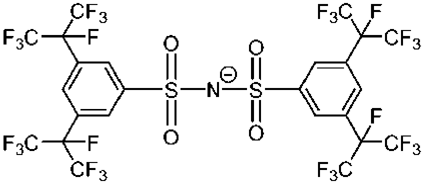 umfasst.

3. Verbindung nach Anspruch 1 oder 2, wobei die Verbindung der Formel (I) keine Verbindungen umfasst, bei denen es sich bei Ligand L um Folgenden handelt:

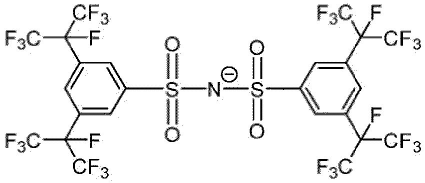

4. Verbindung nach einem der Ansprüche 1 bis 3, wobei mindestens einer von R¹ bis R⁵ und R^{1'} bis R^{5'} für perhalogeniertes C₂- bis C₆-Alkyl steht.

5. Verbindung nach einem der Ansprüche 1 bis 4, wobei mindestens zwei von R¹ bis R⁵ oder R^{1'} bis R^{5'} aus substituiertem C₂- bis C₆-Alkyl ausgewählt sind, wobei der Substituent aus Halogen, Cl, F, CN ausgewählt ist.

6. Verbindung nach den Ansprüchen 1 bis 5, wobei mindestens zwei von R¹ bis R⁵ oder R^{1'} bis R^{5'} aus substituiertem C₁- bis C₆-Alkyl ausgewählt sind, wobei der Substituent aus Halogen, Cl, F, CN ausgewählt sind.

7. Verbindung nach einem der Ansprüche 1 bis 6, wobei M aus einem Alkali-, Erdalkali-, Seltenerd- oder Übergangsmetall, alternativ aus Alkali-, Erdalkali-, Übergangs- oder einem Metall der Gruppe III ausgewählt ist.

8. Organische Halbleiterschicht, wobei die organische Halbleiterschicht eine Verbindung der Formel (I) nach einem der vorhergehenden Ansprüche 1 bis 7 umfasst.

9. Organische elektronische Vorrichtung, umfassend eine Anodenschicht, eine Kathodenschicht und mindestens eine organische Halbleiterschicht, wobei die mindestens eine organische Halbleiterschicht zwischen der Anodenschicht und der Kathodenschicht angeordnet ist und wobei es sich bei der mindestens einen organischen Halbleiterschicht um die organische Halbleiterschicht gemäß Anspruch 8 handelt.

10. Organische elektronische Vorrichtung gemäß Anspruch 9, wobei die Anodenschicht wenigstens eine erste Anoden-Unterschicht und eine zweite Anoden-Unterschicht umfasst.

11. Organische elektronische Vorrichtung nach Anspruch 9 oder 10, wobei die organische elektronische Vorrichtung ferner mindestens eine photoaktive Schicht umfasst, wobei die mindestens eine photoaktive Schicht zwischen der Anodenschicht und der Kathodenschicht angeordnet ist, vorzugsweise die photoaktive Schicht zwischen der organischen Halbleiterschicht und der Kathodenschicht angeordnet ist.

12. Organische elektronische Vorrichtung nach einem der Ansprüche 9 bis 11, wobei es sich bei der photoaktiven Schicht um eine Emissionsschicht handelt.

13. Organische elektronische Vorrichtung nach einem der Ansprüche 9 bis 12, wobei die Vorrichtung mindestens eine Emissionsschicht umfasst, die eine lichtemittierende Verbindung der Formel (IV) 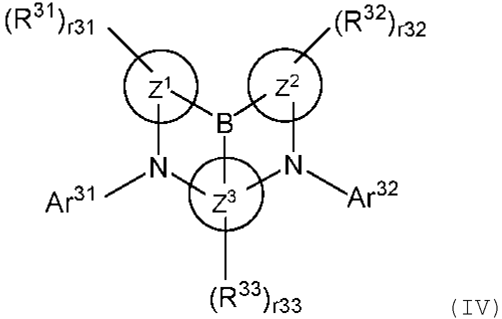 umfasst, wobei
Z¹, Z² und Z³ gleich oder voneinander verschieden sind und jeweils unabhängig aus der Gruppe umfassend einen monocyclischen bis polycyclischen aromatischen Kohlenwasserstoffring oder monocyclischen bis polycyclischen aromatischen Heteroring ausgewählt sind; Ar³¹ und Ar³² gleich sind oder voneinander verschieden ausgewählt sind und jeweils unabhängig für eine substituierte oder unsubstituierte Alkylgruppe, eine substituierte oder unsubstituierte Arylgruppe oder eine substituierte oder unsubstituierte Heteroarylgruppe stehen oder an einen benachbarten Substituenten unter Bildung eines substituierten oder unsubstituierten aromatischen Rings oder eines
substituierten oder unsubstituierten aliphatischen Rings gebunden sind;
R³¹, R³² und R³³ gleich oder voneinander verschieden sind und jeweils unabhängig aus der Gruppe umfassend Wasserstoff, Deuterium, eine substituierte oder unsubstituierte Alkylgruppe, eine substituierte oder unsubstituierte Silylgruppe, eine substituierte oder unsubstituierte Amingruppe, eine substituierte oder unsubstituierte Arylgruppe oder eine substituierte oder unsubstituierte Heteroarylgruppe ausgewählt sind oder benachbarte Substituenten aneinander unter Bildung eines substituierten oder unsubstituierten aromatischen Rings oder eines substituierten oder unsubstituierten aliphatischen Rings gebunden sind,
wobei ein oder mehrere Substituenten, die aus der Gruppe bestehend aus Deuterium, einer Alkylgruppe mit 1 bis 6 Kohlenstoffatomen, einer Alkylsilylgruppe mit 1 bis 30 Kohlenstoffatomen, einer Arylsilylgruppe mit 6 bis 50 Kohlenstoffatomen, einer Alkylamingruppe mit 1 bis 30 Kohlenstoffatomen, einer Alkylarylamingruppe mit 1 bis 50 Kohlenstoffatomen, einer Arylamingruppe mit 6 bis 50 Kohlenstoffatomen, einer Arylgruppe mit 6 bis 30 Kohlenstoffatomen und einer Heteroarylgruppe mit 2 bis 30 Kohlenstoffatomen ausgewählt sind, oder ein Substituent, mit dem zwei oder mehr aus der Gruppe ausgewählte Substituenten verknüpft sind, oder benachbarte Substituenten aneinander unter Bildung eines aliphatischen Kohlenwasserstoffrings mit 3 bis 60 Kohlenstoffatomen, der unsubstituiert oder mit dem Substituenten substituiert ist, gebunden sind; r³¹, r³² und r³³ jeweils für eine ganze Zahl von 0, 1, 2, 3 oder 4 stehen, und wenn r³¹ bis r³³ 2 oder höher sind, Substituenten in der Klammer gleich oder voneinander verschieden sind.

14. Organische elektronische Vorrichtung nach einem der Ansprüche 9 bis 13, wobei es sich bei der organischen elektronischen Vorrichtung um eine Elektrolumineszenzvorrichtung, eine organische Leuchtdiode (OLED), eine lichtemittierende Vorrichtung, einen Dünnschichttransistor, eine Batterie, eine Anzeigevorrichtung oder eine organische photovoltaische Zelle (OPV) handelt.

15. Anzeigevorrichtung, umfassend eine organische elektronische Vorrichtung gemäß einem der Ansprüche 9 bis 14.

## Revendications

1. Composé de formule (1) :
M^{n⊕}(L^{⊖})ₙ (AL)ₘ (I),
M étant un ion métallique ;
n étant la valence de M et choisi parmi 1 à 4 ;
L étant un ligand de formule (II) 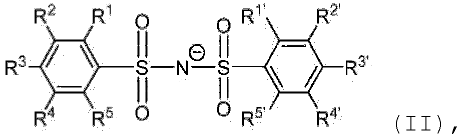
R¹ à R⁵ et R^{1'} à R^{5'} étant indépendamment choisis parmi C₁ à C₆ alkyle substitué ou non substitué, halogène, Cl, F, CN, H ou D ;
au moins un R¹ à R⁵ ou R^{1'} à R^{5'} étant choisi parmi C₂ à C₆ alkyle substitué, le substituant étant choisi parmi halogène, Cl, F, CN ;
AL étant un ligand ancillaire ;
m étant un entier de 0 à 2.

2. Composé selon la revendication 1, le composé de formule (I) ne comprenant pas des composés avec : M = Ag(I) ou Cu(II) et L =

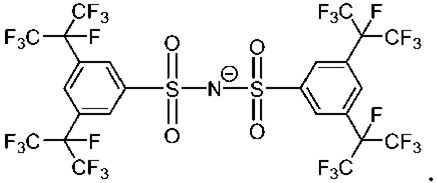

3. Composé selon la revendication 1 ou 2, le composé de formule (I) ne comprenant pas de composés dans lesquels le ligand L est

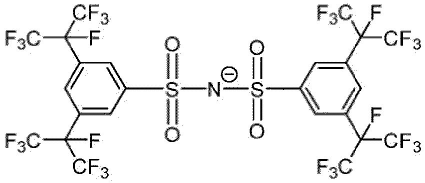

4. Composé selon l'une quelconque des revendications 1 à 3, au moins l'un parmi R¹ à R⁵ et R^{1'} à R^{5'} étant C₂ à C₆ alkyle perhalogéné.

5. Composé selon l'une quelconque des revendications 1 à 4, au moins deux parmi R¹ à R⁵ ou R^{1'} à R^{5'} étant choisis parmi C₂ à C₆ alkyle substitué, le substituant étant choisi parmi halogène, Cl, F, CN.

6. Composé selon les revendications 1 à 5, au moins deux parmi R¹ à R⁵ ou R^{1'} à R^{5'} étant choisis parmi C₁ à C₆ alkyle substitué, le substituant étant choisi parmi halogène, Cl, F, CN.

7. Composé selon l'une quelconque des revendications 1 à 6, M étant choisi parmi un métal alcalin, alcalino-terreux, des terres rares ou de transition, en variante M étant choisi parmi un métal alcalin, alcalino-terreux, de transition ou un métal du groupe III.

8. Couche de semi-conducteur organique, la couche de semi-conducteur organique comprenant un composé de formule (I) selon l'une quelconque des revendications 1 à 7 précédentes.

9. Dispositif électronique organique comprenant une couche d'anode, une couche de cathode et au moins une couche de semi-conducteur organique, l'au moins une couche de semi-conducteur organique étant disposée entre la couche d'anode et la couche de cathode, et l'au moins une couche de semi-conducteur organique étant la couche de semi-conducteur organique selon la revendication 8.

10. Dispositif électronique organique selon la revendication 9, la couche d'anode comprenant au moins une première sous-couche d'anode et une seconde sous-couche d'anode.

11. Dispositif électronique organique selon la revendication 9 ou 10, le dispositif électronique organique comprenant en outre au moins une couche photoactive, l'au moins une couche photoactive étant agencée entre la couche d'anode et la couche de cathode ; de préférence la couche photoactive étant agencée entre la couche de semi-conducteur organique et la couche de cathode.

12. Dispositif électronique organique selon l'une quelconque des revendications 9 à 11, la couche photoactive étant une couche d'émission.

13. Dispositif électronique organique selon l'une quelconque des revendications 9 à 12, le dispositif comprenant au moins une couche d'émission comprenant un composé électroluminescent de formule (IV) 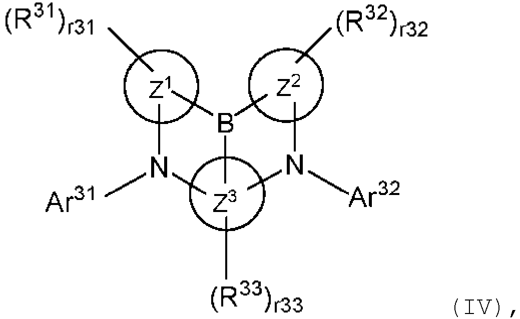
Z¹, Z² et Z³ étant identiques ou différents les uns des autres, et étant chacun indépendamment choisis dans le groupe comprenant un cycle hydrocarboné aromatique monocyclique à polycyclique ou un cycle hétéroaromatique monocyclique à polycyclique ;
Ar³¹ et Ar³² étant identiques ou différents les uns des autres, et étant chacun indépendamment un groupe alkyle substitué ou non substitué, un groupe aryle substitué ou non substitué ou un groupe hétéroaryle substitué ou non substitué, ou étant liés à un substituant adjacent pour former un cycle aromatique substitué ou non substitué ou un
cycle aliphatique substitué ou non substitué ;
R³¹, R³² et R³³ étant identiques ou différents les uns des autres, et étant chacun indépendamment choisis dans le groupe comprenant hydrogène, deutérium, un groupe alkyle substitué ou non substitué, un groupe silyle substitué ou non substitué, un groupe amine substitué ou non substitué, un groupe aryle substitué ou non substitué ou un groupe hétéroaryle substitué ou non substitué, ou des substituants adjacents étant liés les uns aux autres pour former un cycle aromatique substitué ou non substitué ou un cycle aliphatique substitué ou non substitué,
un ou plusieurs substituants choisis dans le groupe constitué par le deutérium, un groupe alkyle ayant de 1 à 6 atomes de carbone, un groupe alkylsilyle ayant de 1 à 30 atomes de carbone, un groupe arylsilyle ayant de 6 à 50 atomes de carbone, un groupe alkylamine ayant de 1 à 30 atomes de carbone, un groupe alkylarylamine ayant de 1 à 50 atomes de carbone, un groupe arylamine ayant de 6 à 50 atomes de carbone, un groupe aryle ayant de 6 à 30 atomes de carbone, et un groupe hétéroaryle ayant de 2 à 30 atomes de carbone, ou un substituant auquel deux ou plusieurs substituants choisis dans le groupe sont liés, ou des substituants adjacents sont liés les uns aux autres pour former un cycle hydrocarboné aliphatique ayant de 3 à 60 atomes de carbone, qui est non substitué ou substitué par le substituant ; r³¹, r³² et r³³ étant chacun un entier de 0, 1, 2, 3 ou 4, et lorsque r³¹ à r³³ sont 2 ou plus, des substituants dans la parenthèse sont les mêmes ou sont différents les uns des autres.

14. Dispositif électronique organique selon l'une quelconque des revendications 9 à 13, le dispositif électronique organique étant un dispositif électroluminescent, une diode émettrice de lumière organique (OLED), un dispositif émetteur de lumière, un transistor à couches minces, une batterie, un dispositif d'affichage ou une cellule photovoltaïque organique (OPV).

15. Dispositif d'affichage comprenant un dispositif électronique organique selon l'une quelconque des revendications 9 à 14.
